(19) Europäisches Patentamt
European Patent Office
Office européen des brevets

(11) **EP 3 580 549 B1**

(12) **EUROPEAN PATENT SPECIFICATION**

(45) Date of publication and mention of the grant of the patent:
**14.07.2021 Bulletin 2021/28**

(21) Application number: **18708910.7**

(22) Date of filing: **09.02.2018**

(51) Int Cl.:
*G01N 21/359* (2014.01)　　*A61K 31/734* (2006.01)
*G01N 21/35* (2014.01)　　*G01N 21/552* (2014.01)

(86) International application number:
**PCT/EP2018/053347**

(87) International publication number:
**WO 2018/146275 (16.08.2018 Gazette 2018/33)**

(54) **A COMPANION DIAGNOSTIC METHOD FOR USE IN THE TREATMENT OF CYSTIC FIBROSIS WITH ALGINATE OLIGOMERS**

BEGLEITENDES DIAGNOSTISCHES VERFAHREN ZUR VERWENDUNG IN DER BEHANDLUNG VON ZYSTISCHER FIBROSE MIT ALGINATOLIGOMEREN

PROCÉDÉ D'ACCOMPAGNEMENT DIAGNOSTIC POUR L'UTILISATION DANS LE TRAITEMENT DE LA FIBROSE KYSTIQUE PAR OLIGOMÈRES D'ALGINATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2017 GB 201702161**

(43) Date of publication of application:
**18.12.2019 Bulletin 2019/51**

(73) Proprietor: **Algipharma AS**
**1337 Sandvika (NO)**

(72) Inventors:
• **LEWIS, Paul**
  **Swansea**
  **West Glamorgan SA2 8PP (GB)**
• **DESSEN, Arne**
  **3440 Røyken (NO)**
• **RYE, Philip**
  **1359 Eiksmarka (NO)**

(74) Representative: **Dehns**
**St. Bride's House**
**10 Salisbury Square**
**London EC4Y 8JD (GB)**

(56) References cited:
• **MANON F. PRITCHARD ET AL: "A New Class of Safe Oligosaccharide Polymer Therapy To Modify the Mucus Barrier of Chronic Respiratory Disease", MOLECULAR PHARMACEUTICS, vol. 13, no. 3, 7 March 2016 (2016-03-07), pages 863-872, XP055327301, US ISSN: 1543-8384, DOI: 10.1021/acs.molpharmaceut.5b00794**
• **D. E. NIVENS ET AL: "Role of Alginate and Its O Acetylation in Formation of Pseudomonas aeruginosa Microcolonies and Biofilms", JOURNAL OF BACTERIOLOGY, vol. 183, no. 3, February 2001 (2001-02), pages 1047-1057, XP055469643, US ISSN: 0021-9193, DOI: 10.1128/JB.183.3.1047-1057.2001**

**Description**

The present invention provides a method to determine the likelihood that a patient with cystic fibrosis (CF) will respond to treatment with an alginate oligomer administered to at least a part of the respiratory system of said patient. In particular the method may be used to predict, or determine the likelihood of, a positive response of the lung/respiratory system of a CF patient to the administration of an alginate oligomer to the lung/respiratory system of the patient, and especially to determine the likelihood of that the alginate oligomer may have a positive (i.e. beneficial) effect on mucus present in the lungs of the CF patient. In this way, it may be determined whether or a not a respiratory complication or symptom of CF in a patient with CF will improve, or the development of said complication or symptom in said patient will be prevented or delayed, following administration of an alginate oligomer to at least part of the respiratory system of the patient. The method of the invention thereby permits the identification of CF patients who are likely to respond clinically to alginate oligomer therapy of their respiratory system. Surprisingly, it has been found that the mucus of the respiratory system (i.e. the heterogeneous gel-like mixture of biomolecules, cells and cell-derived matter found on the mucosal surfaces of the respiratory system), in particular sputum (expectorated mucus of the respiratory system), of CF patients who respond to alginate oligomer therapy of their respiratory system has an infrared (IR) spectrum that is distinguishable from the IR spectrum of respiratory mucus from CF patients that do not respond to alginate oligomers. The method of the present invention is based therefore on analysing the IR absorbance values and/or transmittance values, in particular a Fourier transform infrared spectroscopy (FTIR) spectrum, of a mucus sample from the respiratory system of a CF patient, e.g. a sputum sample, at certain specific wavenumbers.

The term "mucus" is used herein in its broadest sense to refer to the material which coats mucosal surfaces of a mammal. Depending on the location of the mucosal surface in question and/or any pathological processes present at that location or in the host organism as a whole, the constituents of mucus found at that location may vary. At its most basic mucus can be considered to a simple gel-like mixture of mucin glycoproteins and electrolytes secreted by epithelial surfaces. In a physiological setting the mixture is inevitably more complex and other biomaterials, including cells (microbes and those from the host, e.g. inflammatory cells), cell debris, viruses and biomolecules (e.g. proteins, nucleic acids, lipids and polysaccharides) secreted from such cells will be present. In mucus of the respiratory system abiotic particulates (e.g. dust and smoke) may also be present in the mixture. In healthy subjects this heterogeneous mixture of mucin matrix and other biomaterials is efficiently cleared by the normal processes of mucus clearance in the body. In CF patients, however, the basic mucin matrix is itself dysfunctional and therefore inherently resistant to efficient clearance. Reduced clearance means greater amounts of non-mucin components can accumulate in the mucus of CF patients as compared to equivalent mucus in healthy patients and this in turn further exacerbates the difficulties of mucus clearance and can lead to pathological effects from increased microbial colonisation. Thus, the term "mucus" may refer to a material of varying composition depending on the context in which the term is used, but this is entirely consistent with the manner with which the term is used in the art and the skilled person would recognise and understand the use of the term in the context of the present invention to mean essentially the mucous material which may be present on any mucosal surface. In contrast, the term "sputum" is used herein to refer specifically to mucus from the respiratory system which has been expectorated.

CF is an autosomal recessive genetic disease of humans arising from one or more mutations in the cystic fibrosis transmembrane conductance regulator (CFTR) which affect the ability of this ion channel protein to transport chloride and bicarbonate ions across epithelial membranes and thereby also influence the balance of other ions such as sodium. Such mutations can result in insufficient numbers of CFTR at the epithelial cell surface and/or insufficient ion channel activity in the CFTR that are present at the epithelial cell surface (i.e. dysfunctional CFTR ion channels with reduced or absent activity). The perturbations in ionic balance caused by the effects of such mutations in CFTR manifest in stagnant mucus in all organs where mucus is formed and thickened secretions from glands in the liver and the pancreas. The presence of this stagnant mucus in the lungs, paranasal sinuses, gastrointestinal (GI) tract, pancreas, liver and female and male reproductive systems leads to a plethora of clinical conditions associated not only with poor quality of life but also morbidity and mortality. Indeed, most CF sufferers succumb to a complication directly associated with this stagnant, abnormal, mucus.

It has further recently been determined that the problems with mucus in CF patients stem, at least in part, from a failure of the mucus (in particular, some or all of the mucin components) to detach from the underlying mucosa (i.e. from the underlying mucus-secreting epithelium (Gustafsson, J.K., et al (2012) J Exp Med 209, 1263-1272)). Thus, at least to an extent, the abnormal mucus of CF patients is abnormal because it is attached to the underlying epithelium.

In the lungs of CF patients, the dense, attached and intractable mucus is insufficiently cleared by the mucociliary clearance system, and accumulates in the airways. This makes patients susceptible to chronic lung infections and inflammation, which means even greater amounts of bacteria, bacterial biofilm and cell debris become intermixed with the mucus and leads to still greater mucus viscosity. In turn this eventually leads to permanent lung damage and remodelling and further to pulmonary hypertension, heart failure, and respiratory failure. Infection by *Staphylococcus aureus, Haemophilus influenzae, Pseudomonas aeruginosa, Mycobacterium avium complex* and *Aspergillus fumigatus* are common. Abnormal mucus higher up in the respiratory tract (e.g. in the bronchi) can also be susceptible to chronic microbial colonisation which in turn may lead to inflammation of mucosal surfaces (e.g. bronchitis). Response to antibiotics is often poor.

In the paranasal sinuses the abnormal mucus results in frequent blockages leading to facial pain, headaches and abnormal nasal drainage. The sinuses are often exacerbated by infection, to which the abnormal mucus is highly susceptible and this may lead to acute, subacute and chronic sinusitis (also known as rhinosinusitis). Overgrowth of the nasal tissue (nasal polyps) may also result as a consequence of the chronic inflammatory state induced from chronic sinus infection. These polyps can block the nasal passages and increase breathing difficulties.

There is currently no cure for cystic fibrosis, although the life-threatening lung and liver disease can sometimes be resolved with a successful lung or liver transplant. Lung transplants in CF patients are however not always successful because lung infection can recur shortly after transplantation. This is usually a consequence of the use of immunosuppressant drugs to promote establishment of the transplant making the transplant susceptible to the infections that remain in the patient's respiratory system above the newly transplanted lungs.

Pharmaceutical intervention in CF is therefore restricted predominantly to management of secondary symptoms and conditions and very few options are available to address the main underlying cause of those conditions: the abnormal mucus. For instance, lung complications are typically managed through antibiotic, antifungal, anti-inflammatory and bronchodilator treatment regimes. More recently the use of small molecule modulators of the CFTR ion channel, which may regulate ion channel activity, has been proposed and is being clinically tested. Whilst promising, this is still, however, experimental, and other therapies are still being sought and investigated. Alginate oligomers have in this regard also been proposed as a treatment for CF and the complications thereof arising from the abnormal mucus of such patients. The basis of such proposed use is two-fold, firstly the beneficial effect of such oligomers in reducing mucus viscosity, thereby rendering it more readily expellable from the CF lung (WO 2007/039754, WO2008/125828) and secondly the effects of alginate oligomers in assisting in the detachment of the mucus which is abnormally attached to the underlying epithelium (WO 2015/128495).

Alginates are naturally occurring polysaccharides that have been found to have a number of uses, both clinical (e.g. in wound dressings, as drug carriers and in anti-heartburn preparations) and non-clinical (e.g. in food preparation). They are linear polymers of (1-4) linked β-D-mannuronic acid (M) and/or its C-5 epimer α-L-guluronic acid (G). The primary structure of alginates can vary greatly. The M and G residues can be organised as homopolymeric blocks of contiguous M or G residues, as blocks of alternating M and G residues and single M or G residues can be found interspacing these block structures. An alginate molecule can comprise some or all of these structures and such structures might not be uniformly distributed throughout the polymer. In the extreme, there exists a homopolymer of guluronic acid (polyguluronate) or a homopolymer of mannuronic acid (polymannuronate).

Alginates are typically isolated from natural sources such as marine brown algae (e.g. certain species of *Durvillea, Lessonia* and *Laminaria*) and bacteria such as *Pseudomonas aeruginosa* and *Azotobacter vinelandii* as large high molecular weight polymers (e.g. an average molecular weight in the range 300,000 to 500,000 Daltons). It is known, however, that such large alginate polymers may be degraded, or broken down, e.g. by chemical or enzymatic hydrolysis to produce alginate structures of lower molecular weight, e.g. less 35,000 Daltons. It is alginate molecules in this size range (referred to herein and in the art as alginate oligomers) that have been proposed for use in the treatment of CF and other respiratory diseases (see WO 2007/039754, WO 2008/125828 and WO 2015/128495) or to combat biofilm (WO 2009/068841) and multidrug resistant bacteria (WO 2010/139957).

Pritchard, M.F., et al (Molecular Pharmaceutics, 2016, Vol. 13(3), pages 863-872) describes a method of analysing mucin-alginate oligomer (OligoG CF-5/20) interactions by performing ATR-FTIR spectroscopy on sputum samples obtained from cystic fibrosis (CF) patients.

Nivens, D.E., et al (Journal of Bacteriology, 2001, Vol. 183(3), pages 1047-1057) describes a method for investigating the role of alginate and its O Acetylation in the formation of Pseudomonas aeruginosa microcolonies and biofilms based on ATR/FT-IR absorbance spectra obtained as a function of time. One of the samples investigated is a biofilm formed by mucoid CF pulmonary isolate FRD1.

We have now found that not all CF patients respond to therapy with alginate oligomers, and/or that a CF patient may not respond at all times (in other words, that the response of a given CF patient to alginate oligomer therapy may vary from time to time of administration, for example depending on their overall clinical status), e.g. as measured by improvement in the physical properties of the abnormal mucus in their respiratory system or by improvements in their clinical status (e.g. in the respiratory complications/symptoms of CF) upon pulmonary administration of alginate oligomers (that is administration to the respiratory system of said patients). Further investigation has revealed, surprisingly, that the mucus of the respiratory system, in particular sputum, of CF patients who respond (e.g. respond clinically) to pulmonary administration of alginate oligomers has an infrared (IR) spectrum that is distinguishable from the IR spectrum of respiratory mucus from CF patients who do not respond to alginate oligomers. Specifically, said distinguishing IR absorbance or transmittance spectral features are observed at the following infrared (IR) wavenumbers: $1400 \pm 5$ cm$^{-1}$, $1542 \pm 5$ cm$^{-1}$ and $1583 \pm 5$ cm$^{-1}$, that is in the infrared (IR) wavenumber ranges of 1395 cm$^{-1}$ to 1405 cm$^{-1}$, 1537 cm$^{-1}$ to 1547

cm$^{-1}$ and 1578 cm$^{-1}$ to 1588 cm$^{-1}$.

As a first aspect the invention therefore provides a method to determine the likelihood that a CF patient will respond to treatment with an alginate oligomer administered to at least a part of the respiratory system of said patient, said method comprising:

(i) obtaining for a test sample of mucus from the respiratory system of the patient one or more normalised infrared (IR) absorbance values at wavenumbers selected from each of the following wavenumber ranges: 1395 cm$^{-1}$ to 1405 cm$^{-1}$, 1537 cm$^{-1}$ to 1547 cm$^{-1}$ and 1578 cm$^{-1}$ to 1588 cm$^{-1}$; or one or more normalised IR transmittance values at each of said wavenumber ranges, and

(ii) for each of said wavenumber ranges, providing at least one of said absorbance values from that range or at least one of said transmittance values from that range, thereby obtaining a test pattern comprising absorbance and/or transmittance values at each of the three wavenumber ranges and

(iii) comparing the test pattern of absorbance and/or transmittance values so obtained with a negative reference pattern and/or a positive reference pattern, wherein said negative reference pattern is prepared from at least one sample of mucus from the respiratory system of at least one CF patient who did not respond to said treatment, and wherein said positive reference pattern is prepared from at least one sample of mucus from the respiratory system of at least one CF patient who did respond to said treatment, wherein

(a) a test pattern which corresponds to said negative reference pattern and/or which does not correspond to said positive reference pattern is indicative that the patient will not respond to said treatment, and

(b) a test pattern which corresponds to said positive reference pattern and/or which does not correspond with said negative reference pattern is indicative that the patient will respond to said treatment.

A CF patient is a patient with cystic fibrosis (CF).

As noted above, administration to the respiratory system may alternatively be referred to as pulmonary administration, and this is discussed further below.

A response to treatment includes any positive or beneficial response of the patient to the alginate oligomer, and in particular a positive response of the mucus in the respiratory system of the patient (i.e. "respiratory mucus" or "pulmonary mucus"). Thus, the mucus may be rendered less intractable or problematic to the patient, e.g. less stagnant, or less abnormal, most notably by being more susceptible to clearance from the respiratory system by the mucociliary clearance system of the airways (e.g. in the lungs). In other words, there may be a reduction in mucus accumulation. This is discussed further below, but generally this includes a "normalisation" of the CF mucus - the mucus may be rendered more normal, in the sense of more closely resembling non-CF mucus (mucus from a normal, or healthy, subject, or a subject who does not have CF) rather than having the mucus phenotype typically associated with CF. More specifically the mucus may be rendered less viscous and/or it may be detached from the underlying epithelium. A positive response of the respiratory mucus of the CF patient may be manifest as an improvement in the clinical status of the patient (i.e. the patient may respond clinically). Thus, a response to treatment includes an improvement in (e.g. a reduction of), a delay in developing and/or the prevention of one or more clinical parameters of CF, and in particular pulmonary, or respiratory, parameters (that is, particularly, parameters of CF that are manifest in, or detectable in, the lung).

As noted above, CF is associated with a number of complications, or symptoms, of or in the respiratory system of a patient. Accordingly, a response to treatment may include an improvement in a respiratory complication or symptom of CF in the CF patient. Thus, a respiratory complication or symptom of CF may improve (e.g. be reduced), or the development of said complication or symptom in said patient may be prevented or delayed or prevented, following the administration of the alginate oligomer. The alginate oligomer is administered (or delivered) to the respiratory system, which particularly includes the lung, and in particular the administration is such that the mucus in at least a part of the respiratory system of the patient is contacted with an alginate oligomer. This may be termed pulmonary delivery or administration.

Following the method of the invention the alginate oligomer may be administered to the patient if the test pattern for that patient indicates a likelihood that the patient will respond (and conversely, the alginate oligomer would not be administered to the patient if the test pattern for that patient indicates a likelihood that the patient will not respond).

Thus, also described herein is a method of treatment of a CF patient wherein it is first determined by the method defined above that the patient may respond to (i.e. is likely to respond to) administration of an alginate oligomer to the respiratory system, and the method further (or subsequently) comprises administering to the respiratory system of a patient so determined to be a likely responder an effective amount of an alginate oligomer.

The invention therefore provides an alginate oligomer for use in a method of treatment of a CF patient, said method comprising determining that said patient is likely to respond to treatment with said alginate oligomer by performing the method as defined herein and administering to the respiratory system of said patient an effective amount of the alginate oligomer. As defined in more detail below, the "respiratory system" may be taken to include the lungs and all the airways, including the trachea and the nose and nasal passages and throat, through which air may be inhaled or exhaled.

In accordance with the invention, IR absorbance (or transmittance) values may be single values from single wavenumbers or values which are the mean of a plurality of absorbance (transmittance) values obtained at a single (i.e. the same) wavenumber from a wavenumber range of the invention. As such, in accordance with the invention, an IR absorbance (or transmittance) value is not derived from a combination of absorbance (transmittance) values obtained at a plurality of different wavenumbers from a wavenumber range of the invention.

In preferred embodiments the test pattern is formed exclusively from either (i) single IR absorbance values from single wavenumbers from each wavenumber range or mean IR absorbance values from a plurality of absorbance values obtained at the same single wavenumber from each wavenumber range or (ii) single IR transmittance values from single wavenumbers from each wavenumber range or mean IR transmittance values from a plurality of transmittance values obtained at the same single wavenumber from each wavenumber range. In further preferred embodiments the test pattern is formed exclusively from IR absorbance values.

"To determine the likelihood" may be expressed as "to determine the probability" or "to ascertain the probability" or "to assess the probability". As will be clear from the following, the outcomes of these evaluations may be numerical, graphical or illustrative, and in turn each may be qualitative, semi-quantitative or quantitative.

As also described further below, the comparison of step (iii) may be performed virtually (e.g. by software), or mathematically, using an algorithm or mathematical model (e.g. a predictive model) which has been prepared from prior-obtained negative and/or positive reference patterns. Thus, it will clearly be understood that it is not necessary in each case to prepare a positive and/or negative reference pattern (i.e. for each test).

It will also be seen that the method may alternatively be expressed as a method for predicting whether or not a CF patient will respond to treatment with an alginate oligomer (administration of the alginate oligomer to the respiratory system of the patient), e.g. whether or not a respiratory complication or symptom of CF in a CF patient will improve, or is likely to improve, or whether or not development of said respiratory complication in said patient will be delayed or prevented, or is likely to be delayed or prevented, following administration of the alginate oligomer to the respiratory system (or, more specifically, contact of the mucus in at least part of the respiratory system of the patient with an alginate oligomer). The method may also be expressed as a method to identify, detect, diagnose, classify or stratify CF patients who are likely to respond to treatment by administration of an alginate oligomer to the respiratory system. More particularly the method may identify, detect, diagnose, classify or stratify CF patients with a respiratory complication or symptom which will improve, or is likely to improve, or CF patients in which the development of said complication will be delayed or prevented, or is likely to be delayed or prevented, following pulmonary administration of the alginate oligomer (or, more specifically, contact of the mucus in at least part of the respiratory system of the patient with an alginate oligomer).

The invention may also be described as a method for obtaining information useful in, relevant to, or which may assist in, the abovementioned methods.

In certain embodiments the first wavenumber range may be 1396 cm$^{-1}$ to 1404 cm$^{-1}$, 1397 cm$^{-1}$ to 1403 cm$^{-1}$, 1398 cm$^{-1}$ to 1402 cm$^{-1}$, 1399 cm$^{-1}$ to 1401 cm$^{-1}$, or about 1400 cm$^{-1}$. Any other ranges derived from these various range endpoints are specifically contemplated.

In certain embodiments the second wavenumber range may be 1538 cm$^{-1}$ to 1546 cm$^{-1}$, 1539 cm$^{-1}$ to 1545 cm$^{-1}$, 1540 cm$^{-1}$ to 1544 cm$^{-1}$, 1541 cm$^{-1}$ to 1543 cm$^{-1}$, or about 1542 cm$^{-1}$. Any other ranges derived from these various range endpoints are specifically contemplated.

In certain embodiments the third wavenumber range may be 1579 cm$^{-1}$ to 1587 cm$^{-1}$, 1580 cm$^{-1}$ to 1586 cm$^{-1}$, 1581 cm$^{-1}$ to 1585 cm$^{-1}$, 1582 cm$^{-1}$ to 1584 cm$^{-1}$, or about 1583 cm$^{-1}$. Any other ranges derived from these various range endpoints are specifically contemplated.

The method of this aspect of the invention is an *in vitro* method insofar as it is performed on a sample of mucus which has been obtained, or isolated, from the respiratory tract of a patient. In other words the method is not performed *in vivo,* or is not performed on the body of the patient. In more specific aspects the method may further include a step in which the sample is obtained or isolated from the patient, preferably non-surgically or non-invasively. Thus, in this aspect, the treatment of the patient (i.e. the step of administering the alginate oligomer) is not part of the method. Thus, for example, references to outcomes following administration of an alginate oligomer, or contact of the mucus in at least a part of the respiratory system of the patient with an alginate oligomer, are intellectual references to possible outcomes and the step of administering the alginate oligomer, or contacting mucus in at least a part of the respiratory system of the patient with the alginate oligomer, is not part of the methods of this aspect of the invention. However, as will be discussed in more detail further below, it may be the case that alginate oligomers are administered to the patient, or contacted with mucus in at least a part of the respiratory system of a patient, following the method as described in this aspect.

The sample from which IR absorbance or IR transmittance values at the wavenumbers of the invention are obtained may be any sample of mucus from the respiratory system, e.g. those regions recited below. The sample may be obtained by any convenient means, e.g. the endoscopic means recited below, sputum collection and swabbing. It may be advantageous to use a mucus sample which has been obtained from the same part of the respiratory system as that for which it has been established that, following administration of an alginate oligomer, or contact of the mucus with an alginate oligomer, a response to treatment occurs (e.g., an improvement in or delay or prevention of a respiratory complication or symptom of CF occurs). Conveniently, however, the sample is sputum. Sputum collection is a widely practiced technique which simply involves a patient expectorating (or "coughing up") mucus from the lower respiratory tract.

The IR absorbance or IR transmittance values at the wavenumber ranges of the invention may be prepared by any IR (infrared) spectrometer (alternatively referred to as a spectrophotometer, which terms are used interchangeably herein) capable of producing IR light at the required wavenumber range or ranges and measuring IR absorbance and/or IR transmittance. The spectrometer is preferably a portable device, more preferably a hand-held device. Preferably a single device will be used, but this is not essential. In preferred embodiments a Fourier transform infrared (FTIR) spectrometer is used, however devices which measure absorbance or transmittance at monochromatic wavelengths may also be used. In further embodiments the preparation of IR absorbance or IR transmittance values may, at least in part, comprise the use of a transmission or an attenuated total reflection (ATR) technique to apply the IR radiation to the sample. However the invention is by no means limited to the use of ATR-FTIR spectrometers or transmission-FTIR spectrometers and, for instance, a diffuse reflectance infrared Fourier transform (DRIFT) spectrometer may be used to execute a diffuse reflectance FTIR technique. Thus, in one preferred embodiment the IR absorbance and/or transmittance values are FTIR values, and more particularly ATR-FTIR values or transmission FTIR values. Potable and hand-held IR spectrometers, including FTIR spectrometers, are well-known in the art and commercially available from a variety of manufacturers. It may be advantageous to use devices which are configured to use disposable sample containers or strips to which the mucus sample may be applied or collected within.

Step (i) of the method of the invention may further comprise a step in which the one or more normalised IR absorbance or normalised IR transmittance values is prepared from the test sample, e.g. a step in which the test sample is analysed with an IR spectrometer, preferably an FTIR spectrometer, e.g. an ATR- FTIR or a transmission FTIR or a DRIFT spectrometer, and one or more IR absorbance or IR transmittance values at one or more of said wavenumber ranges (preferably all three of said wavenumber ranges) is obtained. These values are then normalised, e.g. as described below.

The preparation of said values will typically involve the subtraction of an internal baseline to allow for the contribution the apparatus and other components of the system make to the values obtained. For example, background readings in the absence of sample can be taken (e.g. a background spectrum can be generated) and may be subtracted from the readings (e.g. spectrum) taken in the presence of sample (i.e. the test or "sample" readings or spectrum). It will be readily apparent that as used herein the term "reference pattern" does not extend to the background pattern, spectrum or reading described immediately above, or other such terms used to define internal baseline readings which allow for the contribution the apparatus and other components of the system make to the test values obtained.

It may further be convenient to prepare said values for a sample from a plurality of individual readings from a single aliquot from the sample, in other words multiple readings at a given or selected wavenumber, or within a given wavenumber range, from a single aliquot from the sample may be obtained. For example, at least 2, preferably at least 3, e.g. at least 4, 5, 6, 8 or 10 readings may be obtained. These values may be used to derive a mean value for a given wavenumber.

In other embodiments at least one reading at a given or selected wavenumber, or within a given wavenumber range, from multiple replicates of the sample are taken. For example, at least one reading (e.g. as described above) from at least 2, preferably at least 3, e.g. at least 4, 5, 6, 8 or 10 replicates may be obtained. These values may be used to derive a mean value for a given wavenumber.

Said individual readings/replicates may be selected (or omitted) on the basis of a similarity (or dissimilarity) measure where only those individual readings/replicates of sufficient similarity (or lack of dissimilarity) are selected. A suitable measure of dissimilarity is the spectral distance (D), i.e. (1 - Pearson's correlation coefficient) x 1000, and in preferred embodiments only those replicates having a D value of equal to or less than 10, e.g. equal to or less than 8, 6, 5, 4, 3, 2 or 1 are selected.

In certain embodiments one or more of the IR absorbance or IR transmittance values at the wavenumber ranges of the invention are provided as part of a continuous IR absorbance (or transmittance) spectrum, or portions thereof, covering one or more of the wavenumber ranges of the invention. Thus, the method may be performed by preparing, or generating, an IR spectrum, e.g. an FTIR spectrum (including a transmission FTIR or an ATR-FTIR or a DRIFT spectrum), for each sample, or each sample replicate, across a wavenumber range which includes the three ranges specified above, for example the range 4000-450 cm$^{-1}$, or a smaller part of this range e.g. 1800-950 cm$^{-1}$ or 1700-1000, or 1700-1200 cm$^{-1}$, etc. Positive and/or negative reference spectra may be analogously prepared.

The IR absorbance or IR transmittance values for the test samples must be normalised to ensure the method is comparative amongst test samples and between the test sample and the positive and negative references. This may be achieved with any convenient statistical means, e.g. Min-Max or vector normalisation. It may be convenient to measure absorbance values (or transmittance values, if appropriate) at a further wavenumber (the "normalisation wavenumber") and then apply a weighting to the test values which is the weighting which must be applied to the value at the further wavenumber to ensure all spectra being compared have equal values at the further wavenumber. The further (normalisation) wavenumber may conveniently be outside the three "test" wavenumber ranges, i.e. is a wavenumber which is not in the three above-noted test ranges. In certain embodiments said further ("normalisation") wavenumber is that which represents the peak of greatest IR absorbance or the least IR transmission, e.g. the Amide I wavenumber, i.e. about 1640 cm$^{-1}$, e.g. 1635 cm$^{-1}$ to 1645 cm$^{-1}$, preferably 1637 cm$^{-1}$ to 1643 cm$^{-1}$ or 1639 cm$^{-1}$ to 1641 cm$^{-1}$.

In certain embodiments the IR absorbance or IR transmittance values are normalised on a scale of 0-2.0 with maximum absorbance (minimum transmission) being set at 2.0 and minimum absorbance (maximum transmission) being set at 0. The skilled person will however be capable of converting such a scale into a scale of alternative dimensions, e.g. a percentage scale may be used, or into an additive inverse (negative) scale.

In accordance with the invention said reference patterns of IR absorbance or IR transmittance values are patterns formed from a combination of absorbance and/or transmittance values at the wavenumber ranges of the invention which is analogous to the pattern of IR absorbance or IR transmittance values from the test sample. In contrast to the test pattern, the reference patterns are obtained from CF patients who have responded clinically to alginate oligomer therapy in their respiratory system (a positive reference pattern) or those which have not (a negative reference pattern). Viewed in the alternative the test pattern should be formed from a combination of absorbance and/or transmittance values at the wavenumber ranges of the invention which is analogous to the pattern of IR absorbance or IR transmittance values from the reference sample(s). Put more simply, the reference patterns may be obtained from one or more (generally a cohort of) responder (positive) and/or one or more (generally a cohort of) non-responder (negative) patients by obtaining normalised IR absorbance and/or transmittance values (e.g. spectra) at the same wavenumber ranges (more particularly the same wavenumbers) as are to be used for the test sample.

More specifically, the reference patterns of IR absorbance and/or IR transmittance are prepared from mucus samples from the respiratory system of CF patients who have responded to pulmonary administration of an alginate oligomer and those who have not. To ensure the most accurate comparison the reference patterns should be prepared in essentially the same way as the test patterns, using the same equipment at the same settings and under the same experimental conditions. However, in practice useful results can still be obtained from different spectrophotometers of the same device resolution so long as equivalent settings and experimental conditions are used.

Thus, for example, a negative reference pattern may be prepared by

(i) obtaining for a negative reference sample of mucus from the respiratory system of a reference CF patient who did not respond to treatment with an alginate oligomer administered to at least a part of the respiratory system one or more normalised IR absorbance values at wavenumbers selected from each of the following wavenumber ranges: 1395 cm$^{-1}$ to 1405 cm$^{-1}$, 1537 cm$^{-1}$ to 1547 cm$^{-1}$ and 1578 cm$^{-1}$ to 1588 cm$^{-1}$; or one or more normalised IR transmittance values at said wavenumber ranges, and
(ii) for each of said wavenumber ranges, providing at least one of said absorbance values from that range or at least one of said transmittance values from that range, thereby obtaining a negative reference pattern of said absorbance values and/or transmittance values, wherein the values provided are analogous to those of the test sample;

and a positive reference pattern may be prepared by

(i) obtaining for a positive reference sample of mucus from the respiratory system of a reference CF patient who did respond to treatment with an alginate oligomer administered to at least a part of the respiratory system one or more normalised IR absorbance values at wavenumbers selected from each of the following wavenumber ranges: 1395 cm$^{-1}$ to 1405 cm$^{-1}$, 1537 cm$^{-1}$ to 1547 cm$^{-1}$ and 1578 cm$^{-1}$ to 1588 cm$^{-1}$; or one or more normalised IR transmittance values at said wavenumber ranges, and
(ii) for each of said wavenumber ranges, providing at least one of said absorbance values from that range or at least one of said transmittance values from that range, thereby obtaining a positive reference pattern of absorbance values and/or transmittance values, wherein the values provided are analogous to those of the test sample.

In other embodiments, average, e.g. mean, reference patterns may be prepared from a plurality of individual reference patterns which have been prepared in essentially the same way and which have an analogous combination of absorbance values and/or transmittance values.

In preferred embodiments the response to treatment which is determined or assessed in accordance with the invention is the same in the test and reference patients. For example, the respiratory complication or symptom of CF which is improved in the CF patient from which the positive reference sample is obtained and/or which is not improved in the CF patient from which the negative reference sample is obtained is the same as the respiratory complication or symptom of CF which is under investigation in the test patient. More particularly, the response to treatment in the reference patients which identifies them (i.e. classifies or designates them) as responders or non-responders is assessed or determined in the same way in the test group.

In further embodiments the mucus samples from the reference CF patients are samples which have been obtained from the same part of the respiratory system as that for which it has been established that, following administration of an alginate oligomer or contact of the mucus in that part of the respiratory system with an alginate oligomer, a response to treatment occurs (e.g. an improvement in or prevention of a respiratory complication or symptom of CF occurs).

In other embodiments the mucus samples from the reference CF patients are from essentially the same part of the respiratory system as the sample from the test patient (or vice versa). In other convenient embodiments the sample is sputum. In further embodiments those reference samples are obtained by the same means and processed and stored in essentially the same way as the test samples (or vice versa).

Comparing the test pattern of IR absorbance and/or IR transmittance values with the reference pattern(s) and assessing correspondence between the two may be a qualitative, semi-quantitative or quantitative process. In the context of the present invention, to correspond to a reference pattern is to be substantially, e.g. essentially, the same as (or to be similar to) a reference pattern. Thus, a test pattern corresponds to a (positive or negative) reference pattern if it is substantially the same, or if it is similar or equivalent to, or matches or fits, a reference pattern e.g. it is statistically similar or statistically equivalent. Thus, the steps of comparing the test and reference patterns and determining whether or not they correspond may be performed using mathematical, or statistical techniques, and generally this will be implemented by software (i.e. it will be performed using a computer). Statistical or mathematical methods for performing such a comparison and determination of correspondence are well known and widely available in the art. It may be determined whether the test pattern is statistically more similar to a positive or to a negative reference pattern to classify, or identify, the patient as a likely responder or non-responder.

In one embodiment IR absorbance and/or IR transmittance spectra, which may be continuous spectra or the relevant portions thereof, are compared visually, or with analogous technologically assisted means, with reference IR absorbance and/or IR transmittance spectra and correspondence between the test spectra and reference spectra at the relevant wavenumber ranges is then estimated. More specifically steps (ii) and (iii) may be expressed as:

(ii) for each of said wavenumber ranges, providing at least one of said absorbance values from that range or at least one of said transmittance values from that range in the form of a test IR spectrum, wherein said IR spectrum is a continuous IR spectrum or portions thereof covering said wavenumber ranges, and

(iii) comparing the test IR spectrum with at least one negative IR reference spectrum and/or at least one positive reference IR spectrum, wherein said at least one negative IR reference spectrum is prepared from at least one sample of mucus from the respiratory system of at least one CF patient who did not respond to said treatment and wherein said positive reference IR spectrum is prepared from at least one sample of mucus from the respiratory system of at least one CF patient who did respond to said treatment,

wherein

(a) a test IR spectrum which corresponds to said negative reference IR spectrum and/or which does not correspond to said positive reference IR spectrum is indicative that the patient will not respond to said treatment (i.e. is a non-responder), and

(b) a test IR spectrum which corresponds to said positive reference IR spectrum and/or which does not correspond to said negative reference IR spectrum is indicative that the patient will respond to said treatment (i.e. is a responder).

The above discussion of the detailed features of reference patterns applies mutatis mutandis to the presently described reference IR spectra.

In another embodiment correspondence between a test and a reference pattern is analysed via representation of the test and reference patterns as single points in Euclidean space having coordinates which are the three values of IR absorbance and/or IR transmittance provided in step (ii). This may result in the provision of a graphical representation of the patterns, e.g. as a 3D scatter plot (graph), but this is not essential. More specifically, steps (ii) and (iii) may be expressed as:

(ii) for each of said wavenumber ranges, providing one of said absorbance values from that range or one of said transmittance values from that range and representing the three so provided values as a single test data point in Euclidean space having coordinates which are the three so provided values (i.e. a single point defined by three dimensions, wherein each of said dimensions represents one of said wavenumber ranges) and

(iii) comparing said test data point with a negative reference data point and/or a positive reference data point, wherein said negative reference data point is prepared from at least one sample of mucus from the respiratory system of at least one CF patient who did not respond to said treatment and wherein said positive reference data point is prepared from at least one sample of mucus from the respiratory system of at least one CF patient who did respond to said treatment,

wherein

(a) collocation of said test data point with said negative reference data point and/or lack of collocation with said positive reference data point is indicative that the patient the patient will not respond to said treatment (i.e. is a non-responder), and
(b) collocation of said test data point with said positive reference data point and/or lack of collocation with said negative reference data point is indicative that the patient will respond to said treatment (i.e. is a responder).

The above discussion of the detailed features of reference patterns applies mutatis mutandis to the presently described reference points.

Collocation of the test data point with a reference data point may be considered to occur if the test data point is within an Euclidean volume defined as having the reference data point at its centre and edges (or boundaries) running in the same three dimensions as the three coordinates of the reference data point, wherein the length of said edges are equal to or less than 50% of the relevant coordinate making up the reference data point (i.e. the absorbance or transmittance value which is in the same dimension as the edge in question). In further embodiments, said edge length is equal to or less than a 40%, e.g. equal to or less than a 30%, 20%, 10% or 2% the relevant coordinate.

In certain embodiments the single test data point is compared to a plurality of reference data points and collocation may be considered to occur if the test data point is substantially, e.g. essentially, at or within the outermost boundaries formed by the cluster of reference points. This may be determined by any convenient cluster analysis technique or algorithm (which may also be referred to as a cluster algorithm, for example hierarchical cluster analysis using Euclidean distance as a distance measure and an 'average' tree building method).

In other embodiments the pattern of test IR absorbance and/or IR transmittance (as appropriate) values can be considered to correspond to a reference pattern if for 2, preferably 3, of the wavenumber ranges of the invention a test IR absorbance (or IR transmittance) value is equal to or less than a 25% positive or negative deviation from the IR absorbance (or IR transmittance) value at the same wavenumber in the same wavenumber range of the reference pattern. In further embodiments, said positive or negative deviation is equal to or less than 20%, e.g. equal to or less than a 15%, 10%, 5% or 1%.

In still further embodiments correspondence between a test and a reference pattern may be analysed by applying said test patterns to a mathematical model generated using the reference pattern(s). Such a mathematical model may be used to determine whether a test pattern fits, or matches, a negative reference pattern or a positive reference pattern, e.g. whether it best fits, or best matches a negative or a positive reference pattern. Mathematical methods for generating such a model are well known, and may be prepared using linear regression techniques. Accordingly, such an analysis may be performed using a linear regression predictive model prepared from a plurality of reference patterns. In specific embodiments the linear regression predictive model may be represented by Formula I or Formula II:

$$\textbf{Formula I:} \quad Prediction\ result = (-1.61 \times A) + (3.07 \times B) + (2.26 \times C) - 5.587$$

wherein A is a normalised IR absorbance value from the wavenumber range 1395 cm$^{-1}$ to 1405 cm$^{-1}$, B is a normalised IR absorbance value from the wavenumber range 1537 cm$^{-1}$ to 1547 cm$^{-1}$, and C is the normalised absorbance value from the wavenumber range 1578 cm$^{-1}$ to 1588 cm$^{-1}$, wherein absorbance is normalised via Min-Max normalisation on a scale of 0 to 2.0 taking the Amide I peak as the reference peak.

In certain embodiments the wavenumber range from which A is derived may be 1396 cm$^{-1}$ to 1404 cm$^{-1}$, 1397 cm$^{-1}$ to 1403 cm$^{-1}$, 1398 cm$^{-1}$ to 1402 cm$^{-1}$, 1399 cm$^{-1}$ to 1401 cm$^{-1}$, or about 1400 cm$^{-1}$. Any other ranges derived from these various range endpoints are specifically contemplated.

In certain embodiments the wavenumber range from which B is derived may be 1538 cm$^{-1}$ to 1546 cm$^{-1}$, 1539 cm$^{-1}$ to 1545 cm$^{-1}$, 1540 cm$^{-1}$ to 1544 cm$^{-1}$, 1541 cm$^{-1}$ to 1543 cm$^{-1}$, or about 1542 cm$^{-1}$. Any other ranges derived from these various range endpoints are specifically contemplated.

In certain embodiments the wavenumber range from which C is derived may be 1579 cm$^{-1}$ to 1587 cm$^{-1}$, 1580 cm$^{-1}$ to 1586 cm$^{-1}$, 1581 cm$^{-1}$ to 1585 cm$^{-1}$, 1582 cm$^{-1}$ to 1584 cm$^{-1}$, or about 1583 cm$^{-1}$. Any other ranges derived from these various range endpoints are specifically contemplated.

A prediction result of 0.5 or above is indicative that the patient will respond to treatment with an alginate oligomer and a prediction result of below 0.5 is indicative that a patient will not respond. The scale used for the normalisation of IR spectroscopy values is arbitrary and a scale of 0-2.0 was used to derive the coefficients in Formula I. As such, should normalisation take place on an alternative scale or use an alternative reference peak, suitable scaling factors may need to be applied to the coefficients in Formula I and the skilled person would be familiar with this.

Should spectra be obtained from, or include, IR transmittance values then conversion of those values to absorbance is required before applying the data to Formula I. The skilled person would be familiar with techniques for achieving this. In one embodiment it may be convenient to apply IR transmittance values to Formula II

**Formula II:** $Prediction\ result = (-1.61 \times (2 - \log_{10}(A*)) + (3.07 \times (2 - \log_{10}(B*)) + (2.26 \times (2 - \log_{10}(C*)) - 5.587$

wherein A* is a normalised % IR transmittance value from the wavenumber range 1395 cm$^{-1}$ to 1405 cm$^{-1}$, B* is a normalised % IR transmittance value from the wavenumber range 1537 cm$^{-1}$ to 1547 cm$^{-1}$, and C* is the normalised % transmittance value from the wavenumber range 1578 cm$^{-1}$ to 1588 cm$^{-1}$, wherein transmittance is normalised via Min-Max normalisation taking the Amide I peak as the reference peak.

In certain embodiments the wavenumber range from which A* is derived may be 1396 cm$^{-1}$ to 1404 cm$^{-1}$, 1397 cm$^{-1}$ to 1403 cm$^{-1}$, 1398 cm$^{-1}$ to 1402 cm$^{-1}$, 1399 cm$^{-1}$ to 1401 cm$^{-1}$, or about 1400 cm$^{-1}$. Any other ranges derived from these various range endpoints are specifically contemplated.

In certain embodiments the wavenumber range from which B* is derived may be 1538 cm$^{-1}$ to 1546 cm$^{-1}$, 1539 cm$^{-1}$ to 1545 cm$^{-1}$, 1540 cm$^{-1}$ to 1544 cm$^{-1}$, 1541 cm$^{-1}$ to 1543 cm$^{-1}$, or about 1542 cm$^{-1}$. Any other ranges derived from these various range endpoints are specifically contemplated.

In certain embodiments the wavenumber range from which C* is derived may be 1579 cm$^{-1}$ to 1587 cm$^{-1}$, 1580 cm$^{-1}$ to 1586 cm$^{-1}$, 1581 cm$^{-1}$ to 1585 cm$^{-1}$, 1582 cm$^{-1}$ to 1584 cm$^{-1}$, or about 1583 cm$^{-1}$. Any other ranges derived from these various range endpoints are specifically contemplated.

In certain embodiments the method of the invention comprises a further, e.g. final, step in which the CF patient is diagnosed as a likely responder (e.g. as having a respiratory complication or symptom of CF which is likely to, preferably will, improve or being a patient in which a respiratory complication would likely be, preferably would be, prevented or delayed from developing upon alginate oligomer therapy in their respiratory system).

The methods of the invention may be performed on a computer, system or apparatus carrying a program adapted to perform said methods. Thus, the methods of the invention may be computer-implemented methods and a computer, system or apparatus may be configured to, e.g. carrying a program adapted to, perform the methods of the invention or certain steps thereof. The system or apparatus may be further adapted to prepare IR absorbance or IR transmittance values at one or more of the wavenumber ranges of the invention, or a step thereof, e.g. the step that results in an IR spectrum of use in accordance with the present invention, preferably in a partially or fully automated manner. The invention thus provides an IR spectrometer (e.g. an FTIR spectrometer), preferably portable or hand-held, carrying a program comprising instructions to cause the infrared spectrometer to perform the methods of the invention described herein. Also described is a computer readable medium carrying said program and such a program per se.

The results (final output) from the methods of the invention may be provided on computer or human readable media or communicated by any suitable means, electronic or otherwise, for comprehension and/or further interpretation by a skilled person.

The method described herein may be followed by a step in which an alginate oligomer is administered to the respiratory system of the CF patient if it is determined that the CF patient is likely to be a responder. The invention therefore provides an alginate oligomer for use in a method of treatment of a CF patient, said method comprising determining that said patient is likely to respond to treatment with said alginate oligomer by performing the method as defined herein and administering to the respiratory system of said patient an effective amount of the alginate oligomer. In preferred embodiments the alginate oligomer administered is the same or substantially the same as an alginate oligomer which has been shown to be effective to treat a CF patient, i.e. on the basis of therapy with which a reference patient has been identified as a responder. Thus, the alginate oligomer may have been shown to improve or prevent or delay a respiratory complication or symptom of CF in a CF patient who is responsive to treatment with alginate oligomers.

Cystic fibrosis is a human disease characterised by mucus and/or exocrine secretions from the lung, pancreas and liver that have abnormal physical properties, typically increased viscosity and, in the case of mucus, adherence to the epithelium of the mucosal surface. These underlying factors manifest in, amongst other conditions, breathing difficulties, respiratory tract infections (chronic and acute, e.g. of the bronchi or of the lungs), respiratory tract inflammation (e.g. bronchiectasis, bronchial inflammation (termed bronchitis, if due to infection) or pulmonary inflammation/pneumonitis (termed pneumonia, if due to infection)), pulmonary hypertension, heart failure, respiratory failure, lung remodelling, sinus infection, sinusitis (acute, subacute and chronic), facial pain, headaches, abnormal nasal drainage, thickened faeces, constipation, bowel obstruction, nutrient malabsorption, pancreatic inflammation, pancreatitis, diabetes, gallstones, liver cirrhosis, and infertility. Decreased response to antibiotics, especially in the lungs, is also seen. The abnormal mucus and exocrine secretions arise from mutations in CFTR which affect the ability of this protein to transport chloride and bicarbonate ions across epithelial membranes and thereby regulate the balance of other ions such as sodium. Many such mutations of CFTR have been identified, some resulting in a more pronounced CF phenotype than others. A subject can therefore be considered to be suffering from CF if the subject has one or more, preferably 2, 3, 4, 5, 6 or more or all of the above mentioned conditions, abnormal mucus (e.g. dense, intractable mucus which, in some instances may be attached to epithelium at at least one mucosal surface), hyperviscous sputum or other secretions and/or exocrine secretions and a mutation in each of his/her CFTR genes.

Conveniently CF may be diagnosed by the "sweat test". This is a routine test familiar to the person skilled in the art. Briefly, pilocarpine is placed on the skin and uptake induced by electric current. Sweat released at the treatment site in response to the pilocarpine is collected (e.g. absorbed onto a piece of filter paper) and is then analysed for its salt content. A person with CF will have salt concentrations that are one-and-one-half to two times greater than normal. More specifically, for infants up to and including 6 months of age, a chloride level of equal to or less than 29 mmol/L means CF is very unlikely; levels of 30 - 59 mmol/L mean that CF is possible; and levels greater than or equal to 60 mmol/L mean CF is likely. For people older than 6 months of age, a chloride level of equal to or less than 39 mmol/L means CF is very unlikely; levels of 40 - 59 mmol/L mean that CF is possible; and levels greater than or equal to 60 mmol/L mean CF is likely.

In accordance with the invention an infant subject (6 months old or younger) to which the treatment of the invention will be applied will have a sweat chloride level of greater than 25 mmol/L, preferably greater than 29mmol/L, 35 mmol/L, 40 mmol/L, 45 mmol/L, 50 mmol/L, 55 mmol/L or 60 mmol/L and all other patients will have a sweat chloride level of greater than 35 mmol/L, preferably greater than 39 mmol/L, 45 mmol/L, 50 mmol/L, 55 mmol/L or 60 mmol/L.

The terms "patient with CF", patient suffering from CF", "patient having CF" and "CF patient" are considered to be equivalent and are used interchangeably herein. The patient may be of any age, e.g. may be a new-born, an infant, a child, a juvenile, an adolescent or an adult.

At its most fundamental a respiratory complication of CF can be considered to be the presence of abnormal mucus (mucus with elevated viscosity and/or which is attached to underlying epithelium) in the respiratory system or part thereof, e.g. the upper respiratory tract (nose, nasal passages, pharynx, larynx and trachea), the paranasal sinuses, and the bronchi (primary, secondary and tertiary) and bronchioles of the lower respiratory tract, in particular in the trachea, bronchi and bronchioles. Thus, an improvement in a respiratory complication following contact with an alginate oligomer may be a decrease in the viscosity of the abnormal mucus in at least a part of the patient's respiratory system and/or a decrease in the attachment of the abnormal mucus to the underlying epithelium of at least a part of the patient's respiratory system. Prevention or delay in these contexts may be expressed as maintaining the viscosity and/or epithelial adherence of respiratory mucus at the level it was prior to treatment with alginate oligomer, or a limitation to the increase in viscosity and/or epithelial adherence that would have occurred had treatment with an alginate oligomer not taken place. In preferred embodiments any decrease in viscosity is effected without a change in the ratio of viscous to elastic response, i.e. the elastic response is decreased by similar proportions. Thus, the mucus may be rendered less intractable or problematic to the patient, e.g. less stagnant, or less abnormal, most notably by being more susceptible to clearance from the respiratory system by the mucociliary clearance system of the airways (e.g. in the lungs). In other words, there may be a reduction in mucus accumulation. Generally this can be seen as a "normalisation" of the CF mucus - the mucus may be rendered more normal, in the sense of more closely resembling non-CF mucus (mucus from a normal, or healthy, subject, or a subject who does not have CF) rather than having the mucus phenotype typically associated with CF.

The properties of the abnormal mucus in the respiratory system of a CF patient manifest typically as reduced mucociliary clearance in the respiratory system. This reduced clearance leads to mucus accumulation. Thus, an improvement in a respiratory complication may be expressed as an increase in mucociliary clearance, or a reduction in mucus accumulation, in at least a part of the patient's respiratory system. Prevention or delay in these contexts may be expressed as maintaining mucociliary clearance, or mucus accumulation, at the level it was prior to treatment with an alginate oligomer, or a limitation to the decrease in mucociliary clearance, or increase in mucus accumulation, that would have occurred had treatment with an alginate oligomer not taken place.

Put more simply, improvement may be considered as restoring the mucus of the patient's respiratory system to a more normal phenotype, e.g. less viscous and/or less adhered, and prevention may be considered as maintaining such normal phenotype. Delay may be considered as any increase in time taken to transition to an abnormal phenotype.

The presence of abnormal mucus in the respiratory system and its subsequent lack of clearance may lead to the development of further complications or symptoms of CF. Such complications or symptoms may be any of those described above, e.g. respiratory tract infections, respiratory tract inflammations, breathing difficulties, respiratory failure, lung remodelling, paranasal sinus infection, sinusitis, facial pain, headaches, abnormal nasal drainage, nasal polyps, and also pulmonary hypertension and heart failure, in which accumulation of mucus and associated bacteria is proposed to contribute to the development of these conditions.

In more specific embodiments the respiratory complication or symptom may be a chronic or an acute infection and/or inflammation in the lower respiratory tract, e.g. in the bronchi or in the lungs, especially chronic infections and/or inflammation. Expressed alternatively, the respiratory complication or symptom may be bronchitis, bronchiectasis or pneumonia. In further specific embodiments the respiratory complication or symptom may be a chronic or an acute infection and/or inflammation in the upper respiratory tract, e.g. in the nose, nasal passages, pharynx, larynx and trachea. Expressed alternatively, the respiratory condition or symptom may be rhinitis (inflammation of the nasal mucosa), nasopharyngitis (or rhinopharyngitis; inflammation of the nasal mucosa, pharynx, hypopharynx, uvula, and tonsils), pharyngitis (inflammation of the pharynx, hypopharynx, uvula, and tonsils), epiglottitis (or supraglottitis; inflammation of the superior portion of the larynx and supraglottic area), laryngitis (inflammation of the larynx), Iryngotracheitis (inflammation of the larynx, trachea, and subglottic area), tracheitis (inflammation of the trachea and subglottic area) and tonsillitis (inflammation of the tonsils). These conditions or symptoms are sometimes collectively termed upper respiratory tract infections.

In certain embodiments it will usually be the case that the respiratory complication or symptom in question will arise from abnormal mucus in the at least part of the respiratory system in question and as such, in these embodiments, the method of the invention will determine the likelihood that treatment with an alginate oligomer administered to at least a part of the respiratory system of a CF patient (or contact of a part of the respiratory system of a CF patient with an alginate oligomer) will improve, delay or prevent a respiratory complication or symptom of CF arising from that part of the patient's respiratory system. For instance, the method of the invention may determine the likelihood that treatment with an alginate oligomer administered to at least a part of the lungs of a CF patient (or contact of mucus in the lungs of a CF patient with the alginate oligomer) will improve, delay or prevent a complication or symptom of CF in the lungs of the CF patient.

"Improvement", when used in relation to these respiratory complications or symptoms, is used broadly herein to include any positive change or reduction in the respiratory complication or symptom in question or indicator thereof. Thus included, for example, is an improvement in any sign of the respiratory complication or symptom, or in any clinically accepted indicator of the respiratory complication or symptom in the patient (for example, decreased respiratory mucus viscosity, decreased mucus adherence to the underlying epithelium of the respiratory system, increased mucociliary clearance in the lungs, reduced mucus accumulation, increased lung function, reduced bioburden in respiratory mucus or, more generally, normalisation of the patient's respiratory mucus). Improvement does not necessarily require the full eradication of the respiratory complication or symptom.

"Delay", when used in relation to these respiratory complications or symptoms, is intended to include any delay, limitation, or reduction to the onset or development of any effect of the respiratory condition or symptom, or one or more indications thereof, or to the onset or development of the respiratory complication or symptom or one or more indications thereof, for example relative to the complication, symptom or indication thereof prior to the treatment.

In contrast "prevention" refers to the absolute prevention of occurrence or development of the respiratory complication or symptom in question, or indication thereof. As CF is a genetic disease which is characterised in each patient by the unique collection of CF-associated complications and symptoms displayed by the patient, the prevention of a respiratory complication or symptom of CF can be considered to be the prevention of any CF-associated respiratory complication or symptom that the patient has yet to acquire or which the patient has acquired previously but has overcome prior to receiving treatment with an alginate oligomer. Prevention can also be considered to include a reduction to the risk of a CF patient acquiring or developing the respiratory complication or symptom in question, or indication thereof.

A further respiratory tract complication is the reduced response to antimicrobials shown by CF patients with an infection in the respiratory system, especially CF patients with a lung infection. Improvement in these contexts may be expressed as an increase in the responsiveness of a CF patient (more specifically the infection in their respiratory system) to antimicrobial agents, e.g. antibiotics, antifungals and antivirals. Prevention in this context may be expressed as maintaining the responsiveness of the patient to an antimicrobial at the level it was prior to treatment with alginate oligomer. Delay in this context may be expressed as any delay or limitation to the reduction in responsiveness to an antimicrobial that would have occurred had treatment with an alginate oligomer not taken place. "Responsiveness to an antimicrobial" is reference to the effects on an infection observed at the patient level for a particular dose of antimicrobial administered in a particular manner. This includes any sign or symptom of the infection observed at the patient level, e.g. microbial load (total or at a specific location, e.g. the lungs), inflammation, fever, microbial toxin levels and general well-being.

It will be further understood that an improvement in a respiratory complication or symptom of CF or the delay or prevention of a respiratory complication or symptom of CF may also be expressed in broad terms as an improvement in CF in said patient. The method of the invention may therefore be considered to be a method to identify CF patients with respiratory complications or symptoms who are likely to or who are not likely to (e.g. will or will not) respond to alginate oligomer therapy of their respiratory system. The method could alternatively be considered to be a method of classifying (or stratifying) CF patients with respiratory complications or symptom in terms of their probable, likely or predicted response to alginate oligomers administered to the respiratory system. The method may further be considered to be a method to identify CF patients in whom the onset or development of a respiratory complication or symptom of CF may be, or is likely to be, delayed or prevented.

In certain embodiments the "improvement" or "delay" in accordance with the invention, or the clinical signs thereof, may be seen within 14 days, e.g. 10 days, 7 days, 5 days, 3 days, 2 days, 1 day 18 hrs, 12hrs or 6 hrs, of commencing treatment with an alginate oligomer in an effective amount.

In certain embodiment the preventive effects of alginate oligomer treatment in accordance with the invention are maintained for the duration of the patient's treatment with an effective amount of alginate oligomer.

It may be the case that a CF patient's status as a patient who is likely to respond to treatment with an alginate oligomer administered to at least part of their respiratory system (as defined in detail herein) may change over time. For instance, CF patients who are determined to be likely responders may become non-responsive over time. Similarly, CF patients who are determined to be likely non-responders may become responsive over time. These transitions between responsive and non-responsive states may occur repeatedly. Accordingly, the methods of the invention may be performed repeatedly in relation to the same patient over time. This may include performing the methods of the invention during or after a patient's treatment with alginate oligomers, e.g. prior to beginning a new course of treatment with an alginate oligomer (in order to confirm likelihood of responsiveness). In other instances the methods of the invention may be performed in relation to a patient after an appropriate period of time following a result showing likely non-responsiveness and/or an unsuccessful course of treatment with an alginate oligomer. In such embodiments the newly generated test patterns from the patient may also be compared (as defined herein) with previous test patterns.

Thus in certain embodiments the CF patient is a patient who has not received treatment with an alginate oligomer administered to at least part of their respiratory system. In other embodiments, the CF patient is a patient undergoing, or who has received, treatment with an alginate oligomer administered to at least part of their respiratory system. The CF patient may be a patient who responded to said treatment or a patient who did not respond to said treatment. In still further embodiments the CF patient is a patient who has previously been identified as a patient who is likely to respond to treatment with an alginate oligomer administered to at least part of their respiratory system or a patient who has previously been identified as a patient who is not likely to respond to said treatment.

As noted above, alginates typically occur as polymers of an average molecular mass of at least 35,000 Daltons, i.e. approximately 175 to approximately 190 monomer residues, and an alginate oligomer may be defined as a material obtained by fractionation (i.e. size reduction) of an alginate polymer, commonly a naturally occurring alginate. An alginate oligomer can therefore be considered to be an alginate of an average molecular weight of less than 35,000 Daltons (i.e. less than approximately 190 or less than approximately 175 monomer residues), in particular an alginate of an average molecular weight of less than 30,000 Daltons (i.e. less than approximately 175 or less than approximately 150 monomer residues) more particularly an average molecular weight of less than 25,000 or 20,000 Daltons (i.e. less than approximately 135 or 125 monomer residues or less than approximately 110 or 100 monomer residues).

Viewed alternatively, an oligomer generally comprises 2 or more monomer units or residues up to the above mentioned size limits and an alginate oligomer according to the invention will typically contain 2 to 100 monomer residues, more typically 3, 4, 5 or 6 to 100, and may contain 2, 3, 4, 5 or 6 to 75, 2, 3, 4, 5 or 6 to 50, 2, 3, 4, 5 or 6 to 40, 2, 3, 4, 5 or 6 to 35 or 2, 3, 4, 5 or 6 to 30 residues. Thus, an alginate oligomer according to the invention will typically have an average molecular weight of 350, 550, 700, 900 or 1000 to 20,000 Daltons, 350, 550, 700, 900 or 1000 to 15,000 Daltons, 350, 550, 700, 900 or 1000 to 10,000 Daltons, 350, 550, 700, 900 or 1000 to 8000 Daltons, 350, 550, 700, 900 or 1000 to 7000 Daltons, or 350, 550, 700, 900 or 1000 to 6,000 Daltons.

Alternatively put, an alginate oligomer may have a degree of polymerisation (DP), or a number average degree of polymerisation (DPn) of 2 to 100, preferably 2 to 75, preferably 2 to 50, more preferably 2 to 40, 2 to 35, 2 to 30, 2 to 28, 2 to 25, 2 to 22, 2 to 20, 2 to 18, 2 to 17, 2 to 15 or 2 to 12.

Other representative ranges (whether for the number of residues, DP or DPn) include any one of 3, 4, 5, 6, 7, 8, 9, 10 or 11 to any one of 50, 45, 40, 39, 38, 37, 36, 35, 34, 33, 32, 31, 30, 29, 28, 27, 26, 25, 24, 23, 22, 21, 20, 19, 18, 17, 16, 15, 14, 13 or 12.

Other representative ranges (whether for the number of residues, DP or DPn) include any one of 8, 9, 10, 11, 12, 13, 14 or 15 to any one of 50, 45, 40, 39, 38, 37, 36, 35, 34, 33, 32, 31, 30, 29, 28, 27, 26, 25, 24, 23, 22, 21, 20, 19, 18, 17 or 16.

Other representative ranges (whether for the number of residues, DP or DPn) include any one of 11, 12, 13, 14, 15, 16, 17 or 18 to any one of 50, 45, 40, 39, 38, 37, 36, 35, 34, 33, 32, 31, 30, 29, 28, 27, 26, 25, 24, 23, 22, 21, 20 or 19.

An alginate oligomer will, as noted above, contain (or comprise) guluronate or guluronic acid (G) and/or mannuronate or mannuronic acid (M) residues or units. An alginate oligomer according to the invention will preferably be composed solely, or substantially solely (i.e. consist essentially of) uronate/uronic acid residues, more particularly solely or substantially solely of G and/or M residues. Alternatively expressed, in an alginate oligomer in accordance with the present invention, at least 80%, more particularly at least 85, 90, 95 or 99% of the monomer residues may be uronate/uronic acid residues, or, more particularly G and/or M residues. In other embodiments an alginate oligomer according to the invention will not comprise other residues or units (e.g. other saccharide residues, or more particularly other uronic acid/uronate residues).

References to "G residues/G" and "M residues/M" or to guluronic acid or mannuronic acid, or guluronate or mannuronate are to be read interchangeably as references to guluronic acid/guluronate and mannuronic acid/mannur-

onate (specifically $\alpha$-L-guluronic acid/guluronate and $\beta$-D-mannuronic acid/mannuronate), and further include derivatives thereof in which one or more available side chains or groups have been modified. Common saccharide modifying groups would include acetyl, sulphate, amino, deoxy, alcohol, aldehyde, ketone, ester and anhydro groups. The alginate oligomers may also be chemically modified to add charged groups (such as carboxylated or carboxymethylated glycans) and to alter flexibility (e.g. by periodate oxidation).

Alginate oligomers may be linear, branched or cyclic.

More particularly, in a preferred embodiment at least 30% of the monomer residues of the alginate oligomer are G residues (i.e. guluronate or guluronic acid). In other words the alginate oligomer will contain at least 30% guluronate (or guluronic acid) residues. Specific embodiments thus include an alginate oligomer with (e.g. containing) 30 to 70% G (guluronate) residues or 70 to 100% G (guluronate) residues. Thus, a representative alginate oligomer according to the present invention may contain at least 70% G residues (i.e. at least 70% of the monomer residues of the alginate oligomer will be G residues).

Preferably at least 50% or 60%, more particularly at least 70% or 75%, even more particularly at least 80, 85, 90, 91, 92, 93, 94, 95, 96, 97, 98 or 99% of the monomer residues are guluronate. In one embodiment the alginate oligomer according to the invention may be an oligoguluronate (i.e. a homooligomer of G, or 100% G)

In a further embodiment, the above described alginate oligomers may have a primary structure wherein the majority of the G residues are in so called G-blocks, e.g. at least 50%, 70%, 75%, 80%, 85%, 90%, 92% or 95% of the G residues may be in G-blocks. A G block is a contiguous sequence of at least two G residues, e.g. at least 3, 4, 5 or 7 contiguous G residues.

In particular at least 90% of the G residues may be linked 1-4 to another G residue. More particularly at least 95%, 98% or 99% of the G residues of the alginate may be linked 1-4 to another G residue. This 1-4 linkage of two G residues can be alternatively expressed as a guluronic unit bound to an adjacent guluronic unit.

Suitable alginate oligomers are described in WO2007/039754, WO2007/039760, WO 2008/125828, WO2009/068841 and WO 2015/128495.

It will thus be seen that a particular class of alginate oligomers according to the present invention is alginate oligomers defined as so-called "high G" or "G-block" oligomers i.e. having a high content of G residues or G-blocks (e.g. wherein at least 70% of the monomer residues are G, preferably arranged in G-blocks). However, other types of alginate oligomer may also be used are also in accordance with the invention, including in particular "high M" or "M-block" oligomers or MG-block oligomers, as described further below. Accordingly, it is alginate oligomers with high proportions of a single monomer type, and with said monomers of this type being present predominantly in contiguous sequences of that monomer type, that represent oligomers that are particularly preferred, e.g. oligomers wherein at least 70% of the monomer residues in the oligomer are G residues linked 1-4 to another G-residue, or more preferably at least 75%, and most preferably at least 80, 85, 90, 92, 93, 94, 95, 96, 97, 98, 99% of the monomers residues of the oligomer are G residues linked 1-4 to another G residue.

In other embodiments at least or, more particularly, more than 50% of the monomer residues of the alginate oligomer according to the invention may be M residues (i.e. mannuronate or mannuronic acid). In other words the alginate oligomer according to the invention may contain at least or, alternatively, more than 50% mannuronate (or mannuronic acid) residues. Specific embodiments thus include alginate oligomers with (e.g. containing) 50 to 70% M (mannuronate) residues or e.g. 70 to 100% M (mannuronate) residues. Further specific embodiments also include oligomers containing 71 to 85% M residues or 85 to 100% M residues. Thus, a representative alginate oligomer according to the present invention will contain more than 70% M residues (i.e. more than 70% of the monomer residues of the alginate oligomer will be M residues).

In other embodiments at least 50% or 60%, more particularly at least 70% or 75%, even more particularly at least 80, 85, 90, 95 or 99% of the monomer residues may be mannuronate. In one embodiment the alginate oligomer may be an oligomannuronate (i.e. a homooligomer of M, or 100%M)

In a further embodiment, the above described alginates according to the invention may have a primary structure wherein the majority of the M residues are in so called M-blocks, e.g. at least 50%, 70%, 75%, 80%, 85%, 90%, 92% or 95% of the M residues are in M-blocks. An M block is a contiguous sequence of at least two M residues, e.g. at least 3, 4, 5 or 7 contiguous M residues.

In particular, at least 90% of the M residues may be linked 1-4 to another M residue. More particularly at least 95%, 98% or 99% of the M residues of the alginate are linked 1-4 to another M residue. This 1-4 linkage of two M residues can be alternatively expressed as a mannuronic unit bound to an adjacent mannuronic unit.

Other oligomers according to the invention are alginate oligomers wherein at least 70% of the monomer residues in the oligomer are M residues linked 1-4 to another M-residue, or more preferably at least 75, 80, 85, 90, 92, 93, 94, 95, 96, 97, 98, 99% of the monomers residues of the oligomer are M residues linked 1-4 to another M residue.

In a still further embodiment, alginate oligomers in accordance with the invention may comprise a sequence of alternating M and G residues. A sequence of at least three, preferably at least four, alternating M and G residues represents an MG block. The alginate oligomers according to the invention may therefore comprise an MG block.

Expressed more specifically, an MG block is a sequence of at least three contiguous residues consisting of G and M residues and wherein each non-terminal (internal) G residue in the contiguous sequence is linked 1-4 and 4-1 to an M residue and each non-terminal (internal) M residue in the contiguous sequence is linked 1-4 and 4-1 to a G residue.

In a further embodiment the minority uronate in the alginate oligomer (i.e. mannuronate or guluronate) may be found predominantly in MG blocks. In this embodiment at least e.g. 50%, 70%, 75%, 80%, 85%, 90% or 95% of the minority uronate monomers in the MG block alginate oligomer are present in MG blocks. In another embodiment the alginate oligomer may be arranged such that at least 50%, 60%, 70%, 80%, 85%, 90%, 95%, or 99%, e.g. 100% of the G and M residues in the oligomer are arranged in MG blocks.

An MG block containing alginate oligomer according to the invention may contain at least 1%, but less than 100%, guluronate (or guluronic acid) residues, but generally an MG block containing alginate oligomer according to the invention will contain at least 30% (or at least 35%, 40%, 45% or 50% G) but less than 100% G. Specific embodiments thus include MG block containing alginate oligomers with (e.g. containing) 1 to 30% G (guluronate) residues, 30 to 70% G (guluronate) residues or 70 to 99% G (guluronate) residues. Thus, a representative MG block containing alginate oligomer according to the present invention may contain more than 30%, but less than 70%, G residues (i.e. more than 30%, but less than 70%, of the monomer residues of the MG block alginate oligomer will be G residues).

More specifically more than 30%, more particularly more than 35%, 40%, 45%, 50%, 55%, 60% or 65%, but in each case less than 70%, of the monomer residues of the MG block containing alginate oligomer according to the invention are guluronate. Alternatively, less than 70%, more particularly less than 65%, 60%, 55%, 50%, 45%, 40% or 35%, but in each case more than 30% of the monomer residues of the MG block containing alginate oligomer according to the invention are guluronate. Any range formed by any combination of these values may be chosen. Therefore for instance the MG block containing alginate oligomer according to the invention can have e.g. between 35% and 65%, 40% and 60% or 45% and 55% G residues.

In another embodiment the MG block containing alginate oligomer according to the invention may have approximately equal amounts of G and M residues (e.g. ratios between 65% G/35% M and 35% G/65% M, for instance 60% G/40% M and 40% G/60% M; 55% G/45% M and 45% G/55% M; 53% G/47% M and 47% G/53% M; 51% G/49% M and 49% G/51% M; e.g. about 50% G and about 50% M) and these residues are arranged predominantly, preferably entirely or as completely as possible, in an alternating MG pattern (e.g. at least 50% or at least 60, 70, 80, 85, 90 or 95% or 100% of the M and G residues are in an alternating MG sequence).

In accordance with the invention a single alginate oligomer or a mixture (multiplicity/plurality) of different alginate oligomers may be administered to (i.e. contacted with the mucus of) at least a part of the respiratory system of the CF patient. Thus, for example, a combination of different alginate oligomers (e.g. two or more) may be administered to (contacted with the mucus of) at least a part of the respiratory system of the CF patient.

Administration of the alginate oligomer (or oligomers) to (i.e. contact with the mucus of) at least a part of the respiratory system of the patient, e.g. in a subsequent treatment step, may be achieved by any convenient means of administering an alginate oligomer to the patient in order to achieve effective amounts thereof in the target part of the respiratory system, more specifically at the mucus layer of the mucosal surface of the target part of the respiratory system, e.g. those recited above. This may be referred to as pulmonary administration.

Such administration may include by topical, enteral (e.g. oral, buccal, sublingual, rectal), parenteral (e.g. intravenous) routes of systemic delivery, but will typically be by inhalation (including nasal inhalation) to achieve localised distribution to the respiratory (pulmonary) system, by which it is meant that delivery is effected to the target part of the respiratory system, but essentially no other location in the patient. The skilled person would be able to select an appropriate administration means to suit any particular respiratory surface he/she is seeking to target, and will be able to formulate alginate oligomers an into pharmaceutical compositions that are adapted for these routes of administration and body distribution according to any of the conventional methods known in the art and widely described in the literature.

More specifically, alginate oligomers may be incorporated, separately or together, optionally together with other active agents, with one or more conventional carriers, diluents and/or excipients, to produce conventional galenic preparations such as tablets, pills, granules (e.g. in free form or enclosed in capsules), powders (e.g. inhalable powders, including dry inhalable powders), lozenges, sachets, cachets, elixirs, suspensions, emulsions, solutions, syrups, aerosols (as a solid or in a liquid medium), sprays (e.g. nasal sprays), compositions for use in nebulisers, ointments, creams, salves, soft and hard gelatine capsules, suppositories, pessaries, sterile injectable solutions, sterile packaged powders, and the like. Sterile inhalable and sterile injectable compositions, especially dry inhalable powders or inhalable solutions, are of particular note.

An "effective amount" of the alginate oligomer is that amount of alginate oligomer that would result in improvement, delay or prevention of a respiratory complication of CF in a CF patient who is clinically responsive to treatment with alginate oligomers, e.g. as defined above.

The skilled man would be able to determine routinely the amount of alginate oligomer he would need to administer to achieve an effective amount. This amount will vary depending on the location of the target treatment (or contact) area, the route of administration and dosage form being used and the particular pharmacokinetic factors that are relevant, but the skilled man would be able to consider all the factors and arrive at a suitable dosing regimen. By way of examples, the alginate oligomer may be used at a daily dose of 0.1g to 10g, e.g. 0.5g to 5g, 0.8g to 3g, 1g to 2g, e.g. about 2g, which may be administered at one or more times per day (e.g. bis daily) and in one or more dosage forms or administration events (e.g. two inhalation events daily).

A representative inhalable powder to be used to administer an alginate oligomer to the lower respiratory tract may contain up to 90%, e.g. up to 85%, 80%, 75% or 70%, e.g. 50 to 90%, 55 to 90%, 60 to 90%, 65 to 90%, 70 to 90%, 75 to 90%, 80 to 90%, 85 to 90%, 50 to 85%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, 75 to 80%, 50 to 70%, 55 to 70%, 60 to 70%, or 65 to 70% w/v or w/w of the oligomer, the remainder being comprised of pharmaceutically acceptable ingredients, e.g. excipients.

A representative inhalable solution to be used to administer an alginate oligomer to the upper respiratory tract typically will be sterile and may contain 6 to 25%, e.g. 6 to 20%, 6 to 15%, 6 to 10%, 8 to 25%, 8 to 20%, 8 to 15 %, 9 to 25%, 9 to 20%, 9 to 15 %, 10 to 15%, 10 to 20%, 10 to 25%, 15 to 20% or 15 to 25% w/v of the oligomer, the remainder being comprised of pharmaceutically acceptable ingredients, e.g. excipients, e.g. water.

For administration to the nose or paranasal sinuses a sterile aqueous and/or oil-based liquid formulation (e.g. an emulsion) may be used; administered for instance by a nasal spray device, e.g. propellant-free or propellant-assisted. A representative formulation may contain 1 to 25%, 1 to 20%, e.g. 1 to 15%, 1 to 10%, 1 to 9%, 1 to 8%, 1 to 7% or 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v or w/w of the oligomer, the remainder being comprised of pharmaceutically acceptable ingredients, e.g. excipients, e.g. water.

A representative solution for intravenous administration of an alginate oligomer to achieve systemic distribution may contain up to 1 to 25%, 1 to 20%, e.g. 1 to 15%, 1 to 10%, 1 to 9%, 1 to 8%, 1 to 7% or 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v or w/w of the alginate oligomer, the remainder being comprised of pharmaceutically acceptable ingredients, e.g. excipients, e.g. water.

A representative oral tablet to be used to administer an alginate oligomer systemically may contain up to 99%, up to 95%, 90%, 85% or 80%, e.g. 50 to 95%, 55 to 95%, 60 to 95%, 65 to 95%, 70 to 95%, 75 to 95%, 80 to 95%, 85 to 95%, 90 to 95%, 50 to 90%, 50 to 90%, 55 to 90%, 60 to 90%, 65 to 90%, 70 to 90%, 75 to 90%, 80 to 90%, 85 to 90%, 50 to 90%, 55 to 85%, 60 to 80% or, 65 to 75% w/v or w/w of the alginate oligomer, the remainder being comprised of pharmaceutically acceptable ingredients, e.g. excipients. The tablet may be a multi-layered tablet.

"% w/v" (or "percentage weight by volume") is a commonly used expression of the concentration of a solid solute in a liquid or semi-solid solvent. 1% w/v equates to 1 gram of solid per 100ml of solvent, 2% w/v equates to 2g of solid per 100ml of solvent, and so on. Accordingly local concentration may be expressed as g/100ml, grams per 100 millilitres, g100ml-1. 1% w/v also equates to 10 gram of solid per litre of solvent. Likewise, "% w/w" (or "percentage weight by weight") is a commonly used expression of the amount of a compound in a solid. 1% w/w equates to 1 gram of compound per 100g of solid, 2% w/w equates to 2g of compound per 100g of solid, and so on. Accordingly, % w/w may be expressed as g/100g, grams per 100 grams and g 100g$^{-1}$. 1% w/w also equates to 10 gram of compound per kilogram of solid. The skilled man would understand that through appropriate scaling calculations, the local concentration range of the present range can be expressed in terms of any SI unit of mass and volume. Conversion into non-standard measures of concentration is also possible and would be routine to the skilled man.

The invention will be further described with reference to the following nonlimiting Examples in which

Figure 1 shows the normalised average IR absorbance spectra within the 1800-950 cm$^{-1}$ IR region for the individual patients of Example 1. Responders: light grey line; Non-responders: dark grey line.

Figure 2 shows a 3D scatterplot of normalised average IR absorbance values at wavenumbers 1583 cm$^{-1}$, 1542 cm$^{-1}$ and 1400cm$^{-1}$ for each patient in Example 1. Responders: solid circles; Non-responders: open circles.

## EXAMPLES

**Example 1** - **Comparative ATR-FTIR analysis of sputum from cystic fibrosis patients who responded to alginate oligomer therapy of their respiratory system and cystic fibrosis patients who did not respond to alginate oligomer treatment therapy of their respiratory system**

1. Methods

1.1. Patients

A double-blind, randomised, placebo-controlled, cross-over study to evaluate the safety, tolerability and preliminary efficacy of alginate oligosaccharide (OligoG, 2600kDa, at least 85% G) administered for 28 days in subjects with Cystic Fibrosis chronically colonised with *Pseudomonas aeruginosa.* All subjects received 4.5mL BID (540mg OligoG or placebo per day) throughout both treatment period 1 and 2. Fourteen patients were selected that had sufficient sputum sample available for the analyses to be performed.

Responder status was defined as those patients showing an observed rheological effect (as determined by Discrete Relaxation Spectrum Analysis of Rheological data) in sputum after OligoG treatment.

Seven patients classed as Responders and seven patients classed as Non-responders were selected for inclusion in this study. Sputum samples obtained from each patient at day 0 of the clinical trial was used in the present study.

*1.2. Fourier Transform IR (FTIR)*

IR spectra were obtained with a Bruker Alpha FTIR instrument equipped with a platinum ATR single reflection diamond-sampling module (Bruker Optics). To control temperature, humidity and prevention of water vapour spectral interference, the instrument was placed in a Captair Pyramid (Erlab) housed in a Concept 1000 workstation (Ruskinn Technology Ltd, Bridgend, Wales). The sampling module was disconnected from the spectrometer and the equipment purged continuously overnight with a gentle flow of $N_2$. For all samples, 3 $\mu$L of sputum was spotted directly onto the ATR sampling module and carefully spread using a pipette tip to ensure that the entire volume covered the sampling window with no spillage. The sample was slowly evaporated at room temperature under a gentle $N_2$ flow to form a film. Infrared spectra were collected as an average of 24 scans per sample between the wavenumber range of 4000-450 $cm^{-1}$ at a resolution of 4 $cm^{-1}$, controlled by Optics User Software (OPUS) version 6.5 (Bruker Optics). The process was repeated until six replicates of set reproducibility were generated per sample. A human genomic DNA spectrum was generated following the same procedure.

Prior to the acquisition of each sample spectrum, the ATR crystal was thoroughly cleaned using isopropyl alcohol in de-ionized water and dried. A background spectrum was then measured using 24 scans and subtracted from the sample spectrum acquired immediately after.

*1.3. Data processing*

The resulting sputum spectra were pre-processed using OPUS software (version 6.5 (Bruker Optics) by subtracting a baseline between 1800 and 950 $cm^{-1}$. The level of reproducibility among the six replicates within each sample was calculated for the 1800-950 $cm^{-1}$ region using the spectral distance (D), a dissimilarity measure, where: D is equal to $(1 - r) \times 1000$ and r is Pearson's correlation coefficient. A spectrum having a D value greater than 10 when compared to any another spectrum would be considered an inadequate replicate and rejected. All spectral comparisons had a D value less than 10 showing high reproducibility. An average spectrum was then calculated for each sample based on the six replicates. Average spectra were normalized using Min-Max normalization and a scale of 0-2.0. This had the effect of absorbance at the Amide 1 peak at approximately 1640 $cm^{-1}$ to be equal across spectra allowing all spectra to be compared.

*1.4. Statistical analysis*

All data analysis was performed using the R Statistical Programming Environment. Significant differences were determined between responder and non-responder group spectra using the Mann-Whitney U test followed by Holm-Bonferroni adjustment of P-values to allow for multiple testing. Correlation between sputum average spectra and the human genomic DNA spectrum was determined using Pearson' correlation. The same procedure was performed to determine correlation between each spectrum and ABS NEUTROPHILS ($x10^9$/L) and NEUTROPHILS (%).

2. Results

*2.1. IR spectra*

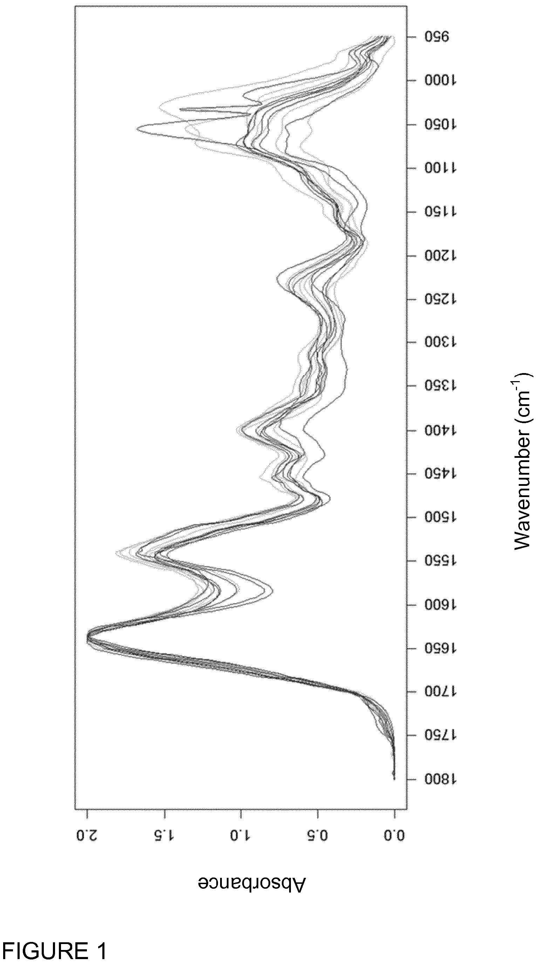

Average spectra within the 'molecular fingerprint' 1800 and 950 $cm^{-1}$ IR region for individual patients were aligned for comparison (Figure 1).

Significant wavenumbers were observed at 1583 $cm^{-1}$, 1542 $cm^{-1}$ and 1400 $cm^{-1}$ (P<0.05 in each case). Wavenumber 1583 $cm^{-1}$ was given the molecular assignment DNA and C=O assym as the molecular bond attributed absorbance. Wavenumber 1542 $cm^{-1}$ was given the molecular assignment Amide II and C=N assym as the molecular bond attributed absorbance. Wavenumber 1400 $cm^{-1}$ was given the molecular assignment DNA and C-N str as the molecular bond attributed absorbance.

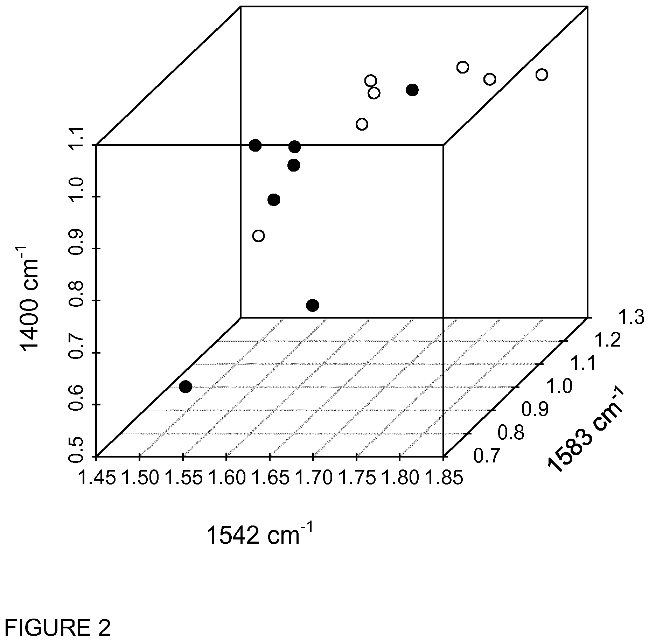

A 3D scatterplot of normalised average IR absorbance values at wavenumbers 1583 cm$^{-1}$, 1542 cm$^{-1}$ and 1400cm$^{-1}$ for each patient is shown in Figure 2. The separation of the two groups is clearly shown is this representation indicating that IR absorbance values at these three wavenumbers may be used together to determine whether a sputum sample is from a patient who is likely to respond to OligoG or from a patient who is not likely to respond to OligoG.

*2.2. Wavenumber model for discrimination of responders*

As an alternative means for discriminating responders from non-responders using IR absorbance values at 1583 cm$^{-1}$, 1542 cm$^{-1}$ and 1400 cm$^{-1}$ a linear regression predictive model was devised based on the normalised IR absorbance data generated as described above.

$$\text{Prediction } [0,1] = (-1.61 \times A) + (3.07 \times B) + (2.26 \times C) - 5.587$$

Where A=normalised absorbance at 1400cm$^{-1}$; B= normalised absorbance at 1542cm$^{-1}$;C= normalised absorbance at 1583cm$^{-1}$; 0 = non-responder and 1 = responder following rounding up of values of equal to or above 0.5 and rounding down of values below 0.5).

**Claims**

1. A method to determine the likelihood that a cystic fibrosis, CF, patient will respond to treatment with an alginate oligomer administered to at least a part of the respiratory system of said patient, said method comprising:

   (i) obtaining for a test sample of mucus from the respiratory system of the patient one or more normalised infrared, IR, absorbance values at wavenumbers selected from each of the following wavenumber ranges: 1395 cm$^{-1}$ to 1405 cm$^{-1}$, 1537 cm$^{-1}$ to 1547 cm$^{-1}$ and 1578 cm$^{-1}$ to 1588 cm$^{-1}$; or one or more normalised IR transmittance values at each of said wavenumber ranges, and
   (ii) for each of said wavenumber ranges, providing at least one of said absorbance values from that range or at least one of said transmittance values from that range, thereby obtaining a test pattern comprising absorbance and/or transmittance values at each of the three wavenumber ranges and
   (iii) comparing the test pattern of absorbance and/or transmittance values so obtained with a negative reference pattern and/or a positive reference pattern, wherein said negative reference pattern is prepared from at least one sample of mucus from the respiratory system of at least one CF patient who did not respond to said treatment, and wherein said positive reference pattern is prepared from at least one sample of mucus from the respiratory system of at least one CF patient who did respond to said treatment, wherein

   (a) a test pattern which corresponds to said negative reference pattern and/or which does not correspond to said positive reference pattern is indicative that the patient will not respond to said treatment, and
   (b) a test pattern which corresponds to said positive reference pattern and/or which does not correspond to said negative reference pattern is indicative that the patient will respond to said treatment.

2. The method of claim 1 wherein said response to treatment comprises an improvement in, the prevention of, or a delay in the development of, a respiratory complication or symptom of CF in the CF patient.

3. The method of claim 1 or claim 2, wherein said test pattern is formed exclusively from either single IR absorbance values from single wavenumbers from each wavenumber range or mean IR absorbance values from a plurality of absorbance values obtained at the same single wavenumber from each wavenumber range.

4. The method of any one of claims 1 to 3, wherein

   (i) the first wavenumber is selected from the range 1396 cm$^{-1}$ to 1404 cm$^{-1}$, 1397 cm$^{-1}$ to 1403 cm$^{-1}$, 1398 cm$^{-1}$ to 1402 cm$^{-1}$, or 1399 cm$^{-1}$ to 1401 cm$^{-1}$, or is 1400 cm$^{-1}$;
   (ii) the second wavenumber is selected from the range 1538 cm$^{-1}$ to 1546 cm$^{-1}$, 1539 cm$^{-1}$ to 1545 cm$^{-1}$, 1540 cm$^{-1}$ to 1544 cm$^{-1}$, or 1541 cm$^{-1}$ to 1543 cm$^{-1}$, or is 1542 cm$^{-1}$; and/or
   (iii) the third wavenumber is selected from the range 1579 cm$^{-1}$ to 1587 cm$^{-1}$, 1580 cm$^{-1}$ to 1586 cm$^{-1}$, 1581 cm$^{-1}$ to 1585 cm$^{-1}$, or 1582 cm$^{-1}$ to 1584 cm$^{-1}$, or is 1583 cm$^{-1}$.

**5.** The method of any one of claims 1 to 4, wherein the sample is sputum.

**6.** The method of any one of claims 1 to 5, wherein the IR absorbance or IR transmittance values are prepared by a Fourier transform infrared, FTIR, spectrometer, preferably an attenuated total reflection-FTIR spectrometer or transmission-FTIR spectrometer.

**7.** The method of any one of claims 1 to 6, wherein one or more of the IR absorbance or IR transmittance values are provided as part of a continuous IR absorbance or transmittance spectrum, respectively, or portions thereof, covering one or more of said wavenumber ranges of the invention, preferably wherein said IR spectrum has the range 4000-450 cm$^{-1}$, 1800-950 cm$^{-1}$, 1700-1000 cm-1, or 1700-1200 cm$^{-1}$.

**8.** The method of any one of claims 1 to 7, wherein the IR absorbance or IR transmittance values are normalised by Min-Max or vector normalisation, preferably wherein normalisation is based on absorbance or transmittance at the Amide I wavenumber, and preferably wherein said Amide I wavenumber is selected from the range 1635 cm$^{-1}$ to 1645 cm$^{-1}$, 1637 cm$^{-1}$ to 1643 cm$^{-1}$, 1639 cm$^{-1}$ to 1641 cm$^{-1}$, or is 1640 cm$^{-1}$.

**9.** The method of any one of claims 1 to 8, wherein steps (ii) and (iii) are:

(ii) for each of said wavenumber ranges, providing at least one of said absorbance values from that range or at least one of said transmittance values from that range in the form of a test IR spectrum, wherein said IR spectrum is a continuous IR spectrum or portions thereof covering said wavenumber ranges, and
(iii) comparing the test IR spectrum with at least one negative IR reference spectrum and/or at least one positive reference IR spectrum, wherein said at least one negative IR reference spectrum is prepared from at least one sample of mucus from the respiratory system of at least one CF patient who did not respond to said treatment and wherein said positive reference IR spectrum is prepared from at least one sample of mucus from the respiratory system of at least one CF patient who did respond to said treatment, wherein

(a) a test IR spectrum which corresponds to said negative reference IR spectrum and/or which does not correspond to said positive reference IR spectrum is indicative that the patient will not respond to said treatment, and
(b) a test IR spectrum which corresponds to said positive reference IR spectrum and/or which does not correspond to said negative reference IR spectrum is indicative that the patient will respond to said treatment.

**10.** The method of any one of claims 1 to 8, wherein steps (ii) and (iii) are:

(ii) for each of said wavenumber ranges, providing one of said absorbance values from that range or one of said transmittance values from that range, and representing the three so provided values as a single test data point in Euclidean space having coordinates which are the three so provided values, and
(iii) comparing said test data point with a negative reference data point and/or a positive reference data point, wherein said negative reference data point is prepared from at least one sample of mucus from the respiratory system of at least one CF patient who did not respond to said treatment and wherein said positive reference data point is prepared from at least one sample of mucus from the respiratory system of at least one CF patient who did respond to said treatment, wherein

(a) collocation of said test data point with said negative reference data point and/or lack of collocation with said positive reference data point is indicative that the patient the patient will not respond to said treatment, and
(b) collocation of said test data point with said positive reference data point and/or lack of collocation with said negative reference data point is indicative that the patient will respond to said treatment.

**11.** The method of any one of claims 1 to 8, wherein the pattern of test IR absorbance and/or IR transmittance values correspond to a reference pattern if for 2, preferably 3, of the wavenumber ranges the test IR absorbance or IR transmittance value is equal to or less than a 25% positive or negative deviation from the IR absorbance or IR transmittance value at the same wavenumber in the same wavenumber range of the reference pattern.

**12.** The method of any one of claims 1 to 8, wherein correspondence between a test and a reference pattern is analysed by applying Formula I:

**Formula I:** $Prediction\ result = (-1.61 \times A) + (3.07 \times B) + (2.26 \times C) - 5.587$

wherein A is a normalised IR absorbance value from the wavenumber range 1395 cm$^{-1}$ to 1405 cm$^{-1}$, B is a normalised IR absorbance value from the wavenumber range 1537 cm$^{-1}$ to 1547 cm$^{-1}$, and C is the normalised absorbance value from the wavenumber range 1578 cm$^{-1}$ to 1588 cm$^{-1}$, wherein absorbance is normalised via Min-Max normalisation on a scale of 0 to 2.0 taking the Amide I peak as the reference peak, and wherein a prediction result of 0.5 or above is indicative that the patient will respond to treatment with an alginate oligomer and a prediction result of below 0.5 is indicative that a patient will not respond.

13. The method of any one of claims 1 to 8, wherein correspondence between a test and a reference pattern is analysed by applying Formula II

**Formula II:** $Prediction\ result = (-1.61 \times (2 - \log_{10}(A*)) + (3.07 \times (2 - \log_{10}(B*)) + (2.26 \times (2 - \log_{10}(C*)) - 5.587$

wherein A* is a normalised % IR transmittance value from the wavenumber range 1395 cm$^{-1}$ to 1405 cm$^{-1}$, B* is a normalised % IR transmittance value from the wavenumber range 1537 cm$^{-1}$ to 1547 cm$^{-1}$, and C* is the normalised % transmittance value from the wavenumber range 1578 cm$^{-1}$ to 1588 cm$^{-1}$, wherein transmittance is normalised via Min-Max normalisation taking the Amide I peak as the reference peak, and wherein a prediction result of 0.5 or above is indicative that the patient will respond to treatment with an alginate oligomer and a prediction result of below 0.5 is indicative that a patient will not respond.

14. The method of any one of claims 1 to 13, wherein the method comprises a further step in which the CF patient is diagnosed as having a respiratory complication or symptom of CF which is likely to improve, or being a patient in which a respiratory complication of CF would likely be prevented or delayed from developing, upon alginate oligomer therapy in their respiratory system.

15. An alginate oligomer for use in a method of treatment of a CF patient, said method comprising determining that said patient is likely to respond to treatment with said alginate oligomer by performing the method as defined in any of claims 1 to 14 and administering to the respiratory system of said patient an effective amount of the alginate oligomer.

16. The method of any one of claims 1 to 14, or the alginate oligomer for use in a method of treatment of a CF patient of claim 15, wherein said CF patient is

(i) a CF patient who has not received treatment with an alginate oligomer administered to at least part of their respiratory system, or
(ii) a CF patient undergoing, or who has received, treatment with an alginate oligomer administered to at least part of their respiratory system.

17. The method of any one of claims 1 to 14 or 16, or the alginate oligomer for use in a method of treatment of a CF patient of claim 15 or 16, wherein the alginate oligomer has

(i) an average molecular weight of less than 35,000 Daltons, preferably less than 30,000, 25,000 or 20,000 Daltons;
(ii) a degree of polymerisation, or a number average degree of polymerisation, of 4 to 100, 4 to 75, 4 to 50, 4 to 35, 4 to 30, 4 to 25, 4 to 22, 4 to 20, 4 to 18, 4 to 16 or 4 to 14;
(iii) a degree of polymerisation, or a number average degree of polymerisation, of 5 to 50, 5 to 35, 5 to 30, 5 to 25, 5 to 22, 5 to 20, 5 to 18, 5 to 16 or 5 to 14,
(iv) a degree of polymerisation, or a number average degree of polymerisation, of 6 to 50, 6 to 35, 6 to 30, 6 to 25, 6 to 22, 6 to 20, 6 to 18, 6 to 16 or 6 to 14, or
(v) a degree of polymerisation, or a number average degree of polymerisation, of 8 to 50, 8 to 35, 8 to 30, 8 to 25, 10 to 25, 10 to 22, 10 to 20, 10 to 18, or 10 to 15.

18. The method of any one of claims 1 to 14, 16 or 17, or the alginate oligomer for use in a method of treatment of a CF patient of any one of claims 15, 16 or 17, wherein the alginate oligomer has

(i) at least 70% guluronate, G, residues, or at least 80%, or at least 85%, or at least 90%, or at least 95% G residues, preferably wherein at least 80% of the G residues of the alginate oligomer are arranged in G-blocks;
(ii) a number average degree of polymerization in the range 5 to 20, a guluronate fraction, $F_G$, of at least 0.85 and a mannuronate fraction, $F_M$, of no more than 0.15; or
(iii) at least 70% mannuronate, M, residues, or at least 80%, or at least 85%, or at least 90%, or at least 95% M residues or 100% M residues, preferably
wherein at least 80% of the M residues of the alginate oligomer are arranged in M-blocks.

19. An infrared spectrometer, preferably portable or hand-held, carrying a computer program comprising instructions to cause the infrared spectrometer to perform a method as claimed in any one of claims 1 to 14 or 16 to 18.

**Patentansprüche**

1. Verfahren zur Bestimmung der Wahrscheinlichkeit, dass ein Patient mit cystischer Fibrose, CF, auf eine Behandlung mit einem Alginat-Oligomer anspricht, das mindestens an einen Teil des Atmungssystems des Patienten verabreicht wird, wobei das Verfahren umfasst:

(i) Erhalten von einem oder mehreren normalisierten Infrarot-, IR-, Absorptionswerten für eine Testprobe von Schleim aus dem Atmungssystem des Patienten bei Wellenzahlen, die aus jedem der folgenden Wellenzahlbereiche ausgewählt sind: 1395 cm$^{-1}$ bis 1405 cm$^{-1}$, 1537 cm$^{-1}$ bis 1547 cm$^{-1}$ und 1578 cm$^{-1}$ bis 1588 cm$^{-1}$; oder einem oder mehreren normalisierten IR-Transmissionswerten bei jedem der genannten Wellenzahlbereiche, und
(ii) für jeden der Wellenzahlbereiche, Bereitstellen von mindestens einem der Absorptionswerte aus diesem Bereich oder mindestens einem der Transmissionswerte aus diesem Bereich, wodurch ein Testmuster erhalten wird, das Absorptions- und/oder Transmissionswerte bei jedem der drei Wellenzahlbereiche umfasst und
(iii) Vergleichen des so erhaltenen Testmusters von Absorptions- und/oder Transmissionswerten mit einem negativen Referenzmuster und/oder einem positiven Referenzmuster, wobei das negative Referenzmuster aus mindestens einer Schleimprobe aus dem Atmungssystem mindestens eines CF-Patienten erstellt wird, der auf die Behandlung nicht angesprochen hat, und wobei das positive Referenzmuster aus mindestens einer Schleimprobe aus dem Atmungssystem mindestens eines CF-Patienten erstellt wird, der auf die Behandlung angesprochen hat,
wobei

(a) ein Testmuster, das dem negativen Referenzmuster entspricht und/oder das nicht dem positiven Referenzmuster entspricht, anzeigt, dass der Patient nicht auf die Behandlung ansprechen wird, und
(b) ein Testmuster, das dem positiven Referenzmuster entspricht und/oder das nicht dem negativen Referenzmuster entspricht, anzeigt, dass der Patient auf die Behandlung ansprechen wird.

2. Verfahren nach Anspruch 1, wobei die Reaktion auf die Behandlung eine Verbesserung, die Verhinderung oder eine Verzögerung der Entwicklung einer Atemwegskomplikation oder eines Symptoms von CF bei dem CF-Patienten umfasst.

3. Verfahren nach Anspruch 1 oder Anspruch 2, wobei das Testmuster ausschließlich entweder aus einzelnen IR-Absorptionswerten von einzelnen Wellenzahlen aus jedem Wellenzahlbereich oder aus mittleren IR-Absorptionswerten aus einer Vielzahl von Absorptionswerten gebildet wird, die bei der gleichen einzelnen Wellenzahl aus jedem Wellenzahlbereich erhalten wurden.

4. Verfahren nach einem der Ansprüche 1 bis 3, wobei

(i) die erste Wellenzahl aus dem Bereich 1396 cm$^{-1}$ bis 1404 cm$^{-1}$, 1397 cm$^{-1}$ bis 1403 cm$^{-1}$, 1398 cm$^{-1}$ bis 1402 cm$^{-1}$ oder 1399 cm$^{-1}$ bis 1401 cm$^{-1}$ ausgewählt ist oder 1400 cm$^{-1}$ ist;
(ii) die zweite Wellenzahl aus dem Bereich 1538 cm$^{-1}$ bis 1546 cm$^{-1}$, 1539 cm$^{-1}$ bis 1545 cm$^{-1}$, 1540 cm$^{-1}$ bis 1544 cm$^{-1}$ oder 1541 cm$^{-1}$ bis 1543 cm$^{-1}$ ausgewählt ist oder 1542 cm$^{-1}$ ist; und/oder
(iii) die dritte Wellenzahl aus dem Bereich 1579 cm$^{-1}$ bis 1587 cm$^{-1}$, 1580 cm$^{-1}$ bis 1586 cm$^{-1}$, 1581 cm$^{-1}$ bis 1585 cm$^{-1}$ oder 1582 cm$^{-1}$ bis 1584 cm$^{-1}$ ausgewählt ist oder 1583 cm$^{-1}$ ist.

5. Verfahren nach einem der Ansprüche 1 bis 4, wobei die Probe Sputum ist.

6. Verfahren nach einem der Ansprüche 1 bis 5, wobei die IR-Absorptions- oder IR-Transmissionswerte mit einem Fourier-Transformations-Infrarot-, FTIR-, Spektrometer, vorzugsweise einem abgeschwächten Totalreflexions-FTIR-Spektrometer oder Transmissions-FTIR-Spektrometer, erstellt werden.

7. Verfahren nach einem der Ansprüche 1 bis 6, wobei einer oder mehrere der IR-Absorptions- oder IR-Transmissionswerte als Teil jeweils eines kontinuierlichen IR-Absorptions- oder Transmissionsspektrums oder Abschnitten davon bereitgestellt werden, das einen oder mehrere der erfindungsgemäßen Wellenzahlbereiche abdeckt, wobei das IR-Spektrum vorzugweise den Bereich 4000-450 cm$^{-1}$, 1800-950 cm$^{-1}$, 1700-1000 cm$^{-1}$ oder 1700-1200 cm$^{-1}$ aufweist.

8. Verfahren nach einem der Ansprüche 1 bis 7, wobei die IR-Absorptions- oder IR-Transmissionswerte durch Min-Max- oder Vektor-Normalisierung normalisiert werden, wobei die Normalisierung vorzugsweise auf der Absorption oder Transmission bei der Amid I-Wellenzahl basiert, und wobei die Amid I-Wellenzahl vorzugsweise aus dem Bereich 1635 cm$^{-1}$ bis 1645 cm$^{-1}$, 1637 cm$^{-1}$ bis 1643 cm$^{-1}$, 1639 cm$^{-1}$ bis 1641 cm$^{-1}$ ausgewählt ist oder 1640 cm$^{-1}$ ist.

9. Verfahren nach einem der Ansprüche 1 bis 8, wobei die Schritte (ii) und (iii) sind:

(ii) für jeden der Wellenzahlbereiche Bereitstellen mindestens eines der Absorptionswerte aus diesem Bereich oder mindestens eines der Transmissionswerte aus diesem Bereich in Form eines Test-IR-Spektrums, wobei das IR-Spektrum ein kontinuierliches IR-Spektrum oder Abschnitte davon ist, das die Wellenzahlbereiche abdeckt, und
(iii) Vergleichen des Test-IR-Spektrums mit mindestens einem negativen IR-Referenzspektrum und/oder mindestens einem positiven Referenz-IR-Spektrum, wobei das mindestens eine negative IR-Referenzspektrum aus mindestens einer Schleimprobe aus dem Atmungssystem mindestens eines CF-Patienten erstellt wird, der auf die Behandlung nicht angesprochen hat, und wobei das positive Referenz-IR-Spektrum aus mindestens einer Schleimprobe aus dem Atmungssystem mindestens eines CF-Patienten erstellt wird, der auf die Behandlung angesprochen hat,
wobei

(a) ein Test-IR-Spektrum, das dem negativen Referenz-IR-Spektrum entspricht und/oder das nicht dem positiven Referenz-IR-Spektrum entspricht, anzeigt, dass der Patient nicht auf die Behandlung ansprechen wird, und
(b) ein Test-IR-Spektrum, das dem positiven Referenz-IR-Spektrum entspricht und/oder das nicht dem negativen Referenz-IR-Spektrum entspricht, anzeigt, dass der Patient auf die Behandlung ansprechen wird.

10. Verfahren nach einem der Ansprüche 1 bis 8, wobei die Schritte (ii) und (iii) sind:

(ii) für jeden der Wellenzahlbereiche, Bereitstellen eines der Absorptionswerte aus diesem Bereich oder eines der Transmissionswerte aus diesem Bereich, und Darstellen der drei so bereitgestellten Werte als einen einzigen Testdatenpunkt im euklidischen Raum, der Koordinaten aufweist, die die drei so bereitgestellten Werte sind, und
(iii) Vergleichen des Testdatenpunkts mit einem negativen Referenzdatenpunkt und/oder einem positiven Referenzdatenpunkt, wobei der negative Referenzdatenpunkt aus mindestens einer Schleimprobe aus dem Atmungssystem von mindestens einem CF-Patienten erstellt wird, der nicht auf die Behandlung ansprach, und wobei der positive Referenzdatenpunkt aus mindestens einer Schleimprobe aus dem Atmungssystem von mindestens einem CF-Patienten erstellt wird, der auf die Behandlung ansprach, wobei

(a) die Kollokation des Testdatenpunkts mit dem negativen Referenzdatenpunkt und/oder das Fehlen einer Kollokation mit dem positiven Referenzdatenpunkt anzeigt, dass der Patient nicht auf die Behandlung ansprechen wird, und
(b) die Kollokation des Testdatenpunkts mit dem positiven Referenzdatenpunkt und/oder das Fehlen einer Kollokation mit dem negativen Referenzdatenpunkt anzeigt, dass der Patient auf die Behandlung ansprechen wird.

11. Verfahren nach einem der Ansprüche 1 bis 8, wobei das Muster der Test-IR-Absorptions- und/oder IR-Transmissionswerte einem Referenzmuster entspricht, wenn für 2, vorzugsweise 3, der Wellenzahlbereiche der Test-IR-Absorptions- oder IR-Transmissionswert gleich oder kleiner als eine 25 %-ige positive oder negative Abweichung von dem IR-Absorptions- oder IR-Transmissionswert bei der gleichen Wellenzahl im gleichen Wellenzahlbereich des Referenzmusters ist.

**12.** Verfahren nach einem der Ansprüche 1 bis 8, wobei die Entsprechung zwischen einem Test- und einem Referenzmuster durch Anwendung der Formel I analysiert wird:

$$\text{Formel I: } \textit{Vorhersageergebnis} = (-1{,}61\text{x}A)+(3{,}07\text{x}B)+(2{,}26\text{x}C)\text{-}5{,}587$$

wobei A ein normalisierter IR-Absorptionswert aus dem Wellenzahlbereich 1395 cm$^{-1}$ bis 1405 cm$^{-1}$ ist, B ein normalisierter IR-Absorptionswert aus dem Wellenzahlbereich 1537 cm$^{-1}$ bis 1547 cm$^{-1}$ ist und C der normalisierte Absorptionswert aus dem Wellenzahlbereich 1578 cm$^{-1}$ bis 1588 cm$^{-1}$ ist, wobei die Absorption mittels Min-Max-Normalisierung auf einer Skala von 0 bis 2,0 normalisiert wird, wobei der Amid I-Peak als Referenzpeak genommen wird, und wobei ein Vorhersageergebnis von 0,5 oder darüber anzeigt, dass der Patient auf die Behandlung mit einem Alginat-Oligomer ansprechen wird, und ein Vorhersageergebnis von unter 0,5 anzeigt, dass ein Patient nicht ansprechen wird.

**13.** Verfahren nach einem der Ansprüche 1 bis 8, wobei die Entsprechung zwischen einem Test- und einem Referenzmuster durch Anwendung der Formel II analysiert wird

$$\text{Formel II: } \textit{Vorhersageergebnis} = (-1{,}61\text{x}(2\text{-}\log_{10}(A*))+(3{,}07\text{x}(2\text{-}\log_{10}(B*))+(2{,}26\text{x}(2\text{-}\log_{10}(C*)))\text{-}5{,}587$$

wobei A* ein normalisierter %-IR-Transmissionswert aus dem Wellenzahlbereich 1395 cm$^{-1}$ bis 1405 cm$^{-1}$ ist, B* ein normalisierter %-IR-Transmissionswert aus dem Wellenzahlbereich 1537 cm$^{-1}$ bis 1547 cm$^{-1}$ ist und C* der normalisierte %-Transmissionswert aus dem Wellenzahlbereich 1578 cm$^{-1}$ bis 1588 cm$^{-1}$ ist, wobei die Transmission mittels Min-Max-Normalisierung unter Verwendung des Amid I-Peaks als Referenzpeak normalisiert wird, und wobei ein Vorhersageergebnis von 0,5 oder darüber anzeigt, dass der Patient auf die Behandlung mit einem Alginat-Oligomer ansprechen wird, und ein Vorhersageergebnis von unter 0,5 anzeigt, dass ein Patient nicht ansprechen wird.

**14.** Verfahren nach einem der Ansprüche 1 bis 13, wobei das Verfahren einen weiteren Schritt umfasst, in dem bei dem CF-Patienten diagnostiziert wird, dass er eine Atemwegskomplikation oder ein Symptom von CF aufweist, die sich wahrscheinlich verbessern werden, oder dass er ein Patient ist, bei dem die Entwicklung einer Atemwegskomplikation von CF wahrscheinlich verhindert oder verzögert werden würde, wenn er eine Alginat-Oligomer-Therapie in seinem Atmungssystem erhält.

**15.** Alginat-Oligomer zur Verwendung in einem Verfahren zur Behandlung eines CF-Patienten, wobei das Verfahren die Bestimmung umfasst, dass der Patient wahrscheinlich auf die Behandlung mit dem Alginat-Oligomer anspricht, indem das Verfahren nach einem der Ansprüche 1 bis 14 durchgeführt wird, und das Verabreichen einer wirksamen Menge des Alginat-Oligomers an das Atmungssystem des Patienten.

**16.** Verfahren nach einem der Ansprüche 1 bis 14 oder das Alginat-Oligomer zur Verwendung in einem Verfahren zur Behandlung eines CF-Patienten nach Anspruch 15, wobei der CF-Patient

(i) ein CF-Patient ist, der keine Behandlung mit einem Alginat-Oligomer erhalten hat, das mindestens an einen Teil seines Atmungssystems verabreicht wurde, oder
(ii) ein CF-Patient ist, der eine Behandlung mit einem Alginat-Oligomer, das mindestens an einen Teil seines Atmungssystems verabreicht wird, durchläuft oder erhalten hat.

**17.** Verfahren nach einem der Ansprüche 1 bis 14 oder 16, oder das Alginat-Oligomer zur Verwendung in einem Verfahren zur Behandlung eines CF-Patienten nach Anspruch 15 oder 16, wobei das Alginat-Oligomer

(i) ein durchschnittliches Molekulargewicht von weniger als 35.000 Dalton, vorzugsweise weniger als 30.000, 25.000 oder 20.000 Dalton, aufweist;
(ii) einen Polymerisationsgrad oder einen Zahlenmittelwert des Polymerisationsgrades von 4 bis 100, 4 bis 75, 4 bis 50, 4 bis 35, 4 bis 30, 4 bis 25, 4 bis 22, 4 bis 20, 4 bis 18, 4 bis 16 oder 4 bis 14 aufweist;
(iii) einen Polymerisationsgrad oder einen Zahlenmittelwert des Polymerisationsgrades von 5 bis 50, 5 bis 35, 5 bis 30, 5 bis 25, 5 bis 22, 5 bis 20, 5 bis 18, 5 bis 16 oder 5 bis 14 aufweist,
(iv) einen Polymerisationsgrad oder einen Zahlenmittelwert des Polymerisationsgrades 6 bis 50, 6 bis 35, 6 bis 30, 6 bis 25, 6 bis 22, 6 bis 20, 6 bis 18, 6 bis 16 oder 6 bis 14 aufweist, oder

(v) einen Polymerisationsgrad einen Zahlenmittelwert des Polymerisationsgrades von 8 bis 50, 8 bis 35, 8 bis 30, 8 bis 25, 10 bis 25, 10 bis 22, 10 bis 20, 10 bis 18 oder 10 bis 15 aufweist.

**18.** Verfahren nach einem der Ansprüche 1 bis 14, 16 oder 17, oder das Alginat-Oligomer zur Verwendung in einem Verfahren zur Behandlung eines CF-Patienten nach einem der Ansprüche 15, 16 oder 17, wobei das Alginat-Oligomer

(i) mindestens 70 % Guluronat-, G-, Reste oder mindestens 80 % oder mindestens 85 % oder mindestens 90 % oder mindestens 95 % G-Reste aufweist, wobei vorzugsweise mindestens 80 % der G-Reste des Alginat-Oligomers in G-Blöcken angeordnet sind;

(ii) einen Zahlenmittelwert des Polymerisationsgrades Polymerisationsgrad im Bereich von 5 bis 20, einen Guluronatanteil, $F_G$, von mindestens 0,85 und einen Mannuronatanteil, $F_M$, von nicht mehr als 0,15 aufweist; oder

(iii) mindestens 70% Mannuronat-, M-, Reste oder mindestens 80% oder mindestens 85 % oder mindestens 90 % oder mindestens 95 % M -Reste oder 100 % M - Reste, wobei vorzugsweise mindestens 80% der M-Reste des Alginatoligomers in M-Blöcken angeordnet sind.

**19.** Infrarotspektrometer, vorzugsweise tragbar oder handgehalten, das ein Computerprogramm trägt, das Anweisungen enthält, um das Infrarotspektrometer zu veranlassen, ein Verfahren nach einem der Ansprüche 1 bis 14 oder 16 bis 18 durchzuführen.

## Revendications

**1.** Procédé pour déterminer la probabilité qu'un patient atteint de fibrose kystique, FK, réponde à un traitement avec un oligomère d'alginate administré à au moins une partie du système respiratoire dudit patient, ledit procédé comprenant :

(i) l'obtention pour un échantillon d'essai de mucus provenant du système respiratoire du patient d'une ou plusieurs valeurs d'absorbance infrarouge, IR, normalisées à des nombres d'onde sélectionnés parmi chacune des plages de nombres d'onde suivantes : 1395 cm$^{-1}$ à 1405 cm$^{-1}$, 1537 cm$^{-1}$ à 1547 cm$^{-1}$ et 1578 cm$^{-1}$ à 1588 cm$^{-1}$; ou d'une ou plusieurs valeurs de transmittance IR normalisées à chacune desdites plages de nombres d'onde, et

(ii) pour chacune desdites plages de nombres d'onde, la fourniture d'au moins une desdites valeurs d'absorbance de cette plage ou d'au moins une desdites valeurs de transmittance de cette plage, obtenant ainsi une séquence de test comprenant des valeurs d'absorbance et/ou de transmittance à chacune des trois plages de nombres d'onde et

(iii) la comparaison de la séquence de test des valeurs d'absorbance et/ou de transmittance ainsi obtenues avec une séquence de référence négative et/ou une séquence de référence positive, dans laquelle ladite séquence de référence négative est préparée à partir d'au moins un échantillon de mucus provenant du système respiratoire d'au moins un patient FK qui n'a pas répondu audit traitement, et dans laquelle ladite séquence de référence positive est préparée à partir d'au moins un échantillon de mucus provenant du système respiratoire d'au moins un patient FK qui a répondu audit traitement,

dans lequel

(a) une séquence de test qui correspond à ladite séquence de référence négative et/ou qui ne correspond pas à ladite séquence de référence positive est l'indication que le patient ne répondra pas audit traitement, et

(b) une séquence de test qui correspond à ladite séquence de référence positive et/ou qui ne correspond pas à ladite séquence de référence négative est l'indication que le patient répondra audit traitement.

**2.** Procédé selon la revendication 1, dans lequel ladite réponse au traitement comprend une amélioration, la prévention, ou un retard dans le développement, d'une complication ou d'un symptôme respiratoire de FK chez le patient FK.

**3.** Procédé selon la revendication 1 ou la revendication 2, dans lequel ladite séquence de test est formée exclusivement à partir soit de valeurs d'absorbance IR uniques provenant de nombres d'onde uniques de chaque plage de nombres d'onde soit de valeurs d'absorbance IR moyennes provenant d'une pluralité de valeurs d'absorbance obtenues au même nombre d'onde unique de chaque plage de nombres d'onde.

**4.** Procédé selon l'une quelconque des revendications 1 à 3, dans lequel

(i) le premier nombre d'onde est sélectionné parmi la plage 1396 cm⁻¹ à 1404 cm⁻¹, 1397 cm⁻¹ à 1403 cm⁻¹, 1398 cm⁻¹ à 1402 cm⁻¹, ou 1399 cm⁻¹ à 1401 cm⁻¹, ou est 1400 cm⁻¹ ;
(ii) le deuxième nombre d'onde est sélectionné parmi la plage 1538 cm⁻¹ à 1546 cm⁻¹, 1539 cm⁻¹ à 1545 cm⁻¹, 1540 cm⁻¹ à 1544 cm⁻¹, ou 1541 cm⁻¹ à 1543 cm⁻¹, ou est 1542 cm⁻¹ ; et/ou
(iii) le troisième nombre d'onde est sélectionné parmi la plage 1579 cm⁻¹ à 1587 cm⁻¹, 1580 cm⁻¹ à 1586 cm⁻¹, 1581 cm⁻¹ à 1585 cm⁻¹, ou 1582 cm⁻¹ à 1584 cm⁻¹, ou est 1583 cm⁻¹.

**5.** Procédé selon l'une quelconque des revendications 1 à 4, dans lequel l'échantillon est une expectoration.

**6.** Procédé selon l'une quelconque des revendications 1 à 5, dans lequel les valeurs d'absorbance IR ou de transmittance IR sont préparées par un spectromètre infrarouge à transformée de Fourier, FTIR, de préférence un spectromètre FTIR par réflexion totale atténuée ou un spectromètre FTIR par transmission.

**7.** Procédé selon l'une quelconque des revendications 1 à 6, dans lequel une ou plusieurs des valeurs d'absorbance IR ou de transmittance IR sont fournies en tant que partie d'un spectre d'absorbance ou de transmittance IR continu, respectivement, ou de portions de celui-ci, couvrant une ou plusieurs desdites plages de nombres d'onde de l'invention, de préférence dans lequel ledit spectre IR présente la plage 4 000-450 cm⁻¹, 1 800-950 cm⁻¹, 1 700-1 000 cm⁻¹, ou 1 700-1 200 cm⁻¹.

**8.** Procédé selon l'une quelconque des revendications 1 à 7, dans lequel les valeurs d'absorbance IR ou de transmittance IR sont normalisées par Min-Max ou par normalisation de vecteur, de préférence dans lequel la normalisation est basée sur l'absorbance ou la transmittance au nombre d'onde de l'Amide I, et de préférence dans lequel ledit nombre d'onde de l'Amide I est sélectionné parmi la plage 1635 cm⁻¹ à 1645 cm⁻¹, 1637 cm⁻¹ à 1643 cm⁻¹, 1639 cm⁻¹ à 1641 cm⁻¹, ou est 1640 cm⁻¹.

**9.** Procédé selon l'une quelconque des revendications 1 à 8, dans lequel les étapes (ii) et (iii) sont :

(ii) pour chacune desdites plages de nombres d'onde, la fourniture d'au moins une desdites valeurs d'absorbance de cette plage ou d'au moins une desdites valeurs de transmittance de cette plage sous la forme d'un spectre IR de test, dans lequel ledit spectre IR est un spectre IR continu ou des portions de celui-ci couvrant lesdites plages de nombres d'onde, et
(iii) la comparaison du spectre IR de test avec au moins un spectre de référence IR négatif et/ou au moins un spectre de référence IR positif, dans laquelle ledit au moins un spectre de référence IR négatif est préparé à partir d'au moins un échantillon de mucus provenant du système respiratoire d'au moins un patient FK qui n'a pas répondu audit traitement et dans laquelle ledit spectre de référence IR positif est préparé à partir d'au moins un échantillon de mucus provenant du système respiratoire d'au moins un patient FK qui a répondu audit traitement,
dans lequel

(a) un spectre IR de test qui correspond audit spectre IR de référence négatif et/ou qui ne correspond pas audit spectre IR de référence positif est l'indication que le patient ne répondra pas audit traitement, et
(b) un spectre IR de test qui correspond audit spectre IR de référence positif et/ou qui ne correspond pas audit spectre IR de référence négatif est l'indication que le patient répondra audit traitement.

**10.** Procédé selon l'une quelconque des revendications 1 à 8, dans lequel les étapes (ii) et (iii) sont :

(ii) pour chacune desdites plages de nombres d'onde, la fourniture d'une desdites valeurs d'absorbance de cette plage ou d'une desdites valeurs de transmittance de cette plage, et la représentation des trois valeurs ainsi fournies en tant que point de données de test unique dans l'espace euclidien présentant des coordonnées qui sont les trois valeurs ainsi fournies, et
(iii) la comparaison dudit point de données de test avec un point de données de référence négatif et/ou un point de données de référence positif, dans laquelle ledit point de données de référence négatif est préparé à partir d'au moins un échantillon de mucus provenant du système respiratoire d'au moins un patient FK qui n'a pas répondu audit traitement et dans laquelle ledit point de données de référence positif est préparé à partir d'au moins un échantillon de mucus provenant du système respiratoire d'au moins un patient FK qui a répondu audit traitement, dans lequel

(a) une collocation dudit point de données de test avec ledit point de données de référence négatif et/ou une absence de collocation avec ledit point de données de référence positif est l'indication que le patient ne répondra pas audit traitement, et

(b) une collocation dudit point de données de test avec ledit point de données de référence positif et/ou une absence de collocation avec ledit point de données de référence négatif est l'indication que le patient répondra audit traitement.

11. Procédé selon l'une quelconque des revendications 1 à 8, dans lequel la séquence de valeurs d'absorbance IR et/ou de transmittance IR de test correspond à une séquence de référence si pour 2, de préférence 3, des plages de nombres d'onde la valeur d'absorbance IR ou de transmittance IR de test est égale ou inférieure à un écart positif ou négatif de 25 % par rapport à la valeur d'absorbance IR ou de transmittance IR au même nombre d'onde dans la même plage de nombres d'onde de la séquence de référence.

12. Procédé selon l'une quelconque des revendications 1 à 8, dans lequel une correspondance entre une séquence de test et une séquence de référence est analysée en appliquant la Formule I :

$$\text{Formule I}: \quad R\acute{e}sultat\ de\ pr\acute{e}diction = (-1{,}61 \times A) + (3{,}07 \times B) + (2{,}26 \times C) - 5{,}587$$

dans laquelle A est une valeur d'absorbance IR normalisée provenant de la plage de nombres d'onde 1395 cm$^{-1}$ à 1405 cm$^{-1}$, B est une valeur d'absorbance IR normalisée provenant de la plage de nombres d'onde 1537 cm$^{-1}$ à 1547 cm$^{-1}$, et C est la valeur d'absorbance normalisée provenant de la plage de nombres d'onde 1578 cm$^{-1}$ à 1588 cm$^{-1}$, dans laquelle l'absorbance est normalisée par normalisation Min-Max sur une échelle de 0 à 2,0 en prenant le pic d'Amide I comme le pic de référence, et dans laquelle un résultat de prédiction de 0,5 ou plus est l'indication que le patient répondra au traitement avec un oligomère d'alginate et un résultat de prédiction inférieur à 0,5 est l'indication que le patient ne répondra pas.

13. Procédé selon l'une quelconque des revendications 1 à 8, dans lequel une correspondance entre une séquence de test et une séquence de référence est analysée en appliquant la Formule II

$$\text{Formule II}: \quad R\acute{e}sultat\ de\ pr\acute{e}diction = (-1{,}61 \times (2 - \log_{10}(A*)) + (3{,}07 \times (2 - \log_{10}(B*)) + (2{,}26 \times (2 - \log_{10}(C*)) - 5{,}587$$

dans laquelle A* est une valeur de transmittance IR en % normalisée provenant de la plage de nombres d'onde 1395 cm$^{-1}$ à 1405 cm$^{-1}$, B* est une valeur de transmittance IR en % normalisée provenant de la plage de nombres d'onde 1537 cm$^{-1}$ à 1547 cm$^{-1}$, et C* est la valeur de transmittance en % normalisée provenant de la plage de nombres d'onde 1578 cm$^{-1}$ à 1588 cm$^{-1}$, dans laquelle la transmittance est normalisée par normalisation Min-Max en prenant le pic d'Amide I comme le pic de référence, et dans laquelle un résultat de prédiction de 0,5 ou plus est l'indication que le patient répondra au traitement avec un oligomère d'alginate et un résultat de prédiction inférieur à 0,5 est l'indication que le patient ne répondra pas.

14. Procédé selon l'une quelconque des revendications 1 à 13, dans lequel le procédé comprend une étape supplémentaire dans laquelle le patient FK est diagnostiqué comme présentant une complication ou un symptôme respiratoire de FK qui est susceptible de s'améliorer, ou comme étant un patient chez qui une complication respiratoire de FK serait susceptible d'être évitée ou retardée de se développer, avec une thérapie par oligomère d'alginate dans son système respiratoire.

15. Oligomère d'alginate pour une utilisation dans un procédé de traitement d'un patient FK, ledit procédé comprenant la détermination que ledit patient est susceptible de répondre au traitement avec ledit oligomère d'alginate en mettant en œuvre le procédé tel que défini dans l'une quelconque des revendications 1 à 14 et l'administration au système respiratoire dudit patient d'une quantité efficace de l'oligomère d'alginate.

16. Procédé selon l'une quelconque des revendications 1 à 14, ou oligomère d'alginate pour une utilisation dans un procédé de traitement d'un patient FK selon la revendication 15, dans lequel ledit patient FK est

(i) un patient FK qui n'a pas reçu de traitement avec un oligomère d'alginate administré à au moins une partie de son système respiratoire, ou

(ii) un patient FK qui suit, ou qui a reçu, un traitement avec un oligomère d'alginate administré à au moins une partie de son système respiratoire.

**17.** Procédé selon l'une quelconque des revendications 1 à 14 ou 16, ou oligomère d'alginate pour une utilisation dans un procédé de traitement d'un patient FK selon la revendication 15 ou 16, dans lequel l'oligomère d'alginate présente

(i) un poids moléculaire moyen inférieur à 35 000 Daltons, de préférence inférieur à 30 000, 25 000 ou 20 000 Daltons ;

(ii) un degré de polymérisation, ou un degré de polymérisation moyen en nombre, de 4 à 100, 4 à 75, 4 à 50, 4 à 35,4 à 30, 4 à 25,4 à 22, 4 à 20,4 à 18, 4 à 16 ou 4 à 14;

(iii) un degré de polymérisation, ou un degré de polymérisation moyen en nombre, de 5 à 50, 5 à 35, 5 à 30, 5 à 25, 5 à 22, 5 à 20, 5 à 18, 5 à 16 ou 5 à 14,

(iv) un degré de polymérisation, ou un degré de polymérisation moyen en nombre, de 6 à 50, 6 à 35, 6 à 30, 6 à 25, 6 à 22, 6 à 20, 6 à 18, 6 à 16 ou 6 à 14, ou

(v) un degré de polymérisation, ou un degré de polymérisation moyen en nombre, de 8 à 50, 8 à 35, 8 à 30, 8 à 25, 10 à 25, 10 à 22, 10 à 20, 10 à 18, ou 10 à 15.

**18.** Procédé selon l'une quelconque des revendications 1 à 14, 16 ou 17, ou oligomère d'alginate pour une utilisation dans un procédé de traitement d'un patient FK selon l'une quelconque des revendications 15, 16 ou 17, dans lequel l'oligomère d'alginate présente

(i) au moins 70 % de résidus de guluronate, G, ou au moins 80 %, ou au moins 85 %, ou au moins 90 %, ou au moins 95 % de résidus G, de préférence dans lesquels au moins 80 % des résidus G de l'oligomère d'alginate sont disposés en blocs G ;

(ii) un degré de polymérisation moyen en nombre dans la plage 5 à 20, une fraction de guluronate, FG, d'au moins 0,85 et une fraction de mannuronate, FM, ne dépassant pas 0,15 ; ou

(iii) au moins 70% de résidus de mannuronate, M, ou au moins 80 %, ou au moins 85 %, ou au moins 90 %, ou au moins 95 % de résidus M ou 100 % de résidus M, de préférence dans lesquels au moins 80 % des résidus M de l'oligomère d'alginate sont disposés en blocs M.

**19.** Spectromètre infrarouge, de préférence portable ou portatif, comportant un programme informatique comprenant des instructions pour amener le spectromètre infrarouge à mettre en œuvre un procédé selon l'une quelconque des revendications 1 à 14 ou 16 à 18.

FIGURE 1

FIGURE 2

**REFERENCES CITED IN THE DESCRIPTION**

**Patent documents cited in the description**

- WO 2007039754 A
- WO 2008125828 A
- WO 2015128495 A
- WO 2009068841 A
- WO 2010139957 A
- WO 2007039760 A

**Non-patent literature cited in the description**

- **GUSTAFSSON, J.K. et al.** *J Exp Med,* 2012, vol. 209, 1263-1272
- **PRITCHARD, M.F. et al.** *Molecular Pharmaceutics,* 2016, vol. 13 (3), 863-872
- **NIVENS, D.E. et al.** *Journal of Bacteriology,* 2001, vol. 183 (3), 1047-1057